# EUROPEAN PATENT SPECIFICATION

(11) **EP 2 329 821 B1**
(45) Date of publication and mention of the grant of the patent: **22.08.2012**
(21) Application number: 10170798.2
(22) Date of filing: 29.11.2006
(51) Int. Cl.: A61K 31/506, A61P 27/02, A61K 31/55, A61K 31/7052, A61K 39/395

(54) **Treatment of ocular neovascular disorders such as macular degeneration, angiod streaks, uveitis and macular edema**
Behandlung von neovaskulären Augenerkrankungen, wie z.B. Maculadegeneration, angioiden Streifen, Uveitis und Makulaödemen
Traitement de troubles neovasculaires oculaires comme par example dègèneresence maculaire, uveite et oedème maculaire

(30) Priority: 29.11.2005 US 740478 P
(43) Date of publication of application: 08.06.2011
(62) Divisional of application: 06838635.8
(73) Proprietor: GlaxoSmithKline LLC, Philadelphia, PA 19102 (US)
(72) Inventor: Brigandi, Richard Anthony, Collegeville, PA 19426 (US); Levick, Mark, Collegeville, PA 19426 (US); Miller, William Henry, Collegeville, PA 19426 (US)
(74) Representative: Learoyd, Stephanie Anne

(56) References cited:
- WO-A-02/059110
- WO-A-03/106416
- WO-A-2006/117666
- HOYNG C B ET AL: "AG-013958 (VEGFR inhibitor) achieves effective choroidal concentrations withminimal systemic effects in cynomolgus monkeys and humans with age-related macular disease", INVESTIGATIVE OPHTHALMOLOGY & VISUAL SCIENCE, ASSOCIATION FOR RESEARCH IN VISION AND, US, vol. 46, no. Suppl S, May 2005 (2005-05), page 2365, XP009096897, ISSN: 0146-0404
- KHALIL D ET AL: "Nonclinical ADME studies of AG-013958 demonstrate sustained ocular exposure and low systemic exposure", INVESTIGATIVE OPHTHALMOLOGY & VISUAL SCIENCE, ASSOCIATION FOR RESEARCH IN VISION AND, US, vol. 46, no. Suppl S, May 2005 (2005-05), page 5378, XP009096896, ISSN: 0146-0404
- GALE D C ET AL: "Ocular pharmacokinetics in single compound and cassette dose studies following sub-Tenon administration in Dutch-belted rabbits", INVESTIGATIVE OPHTHALMOLOGY & VISUAL SCIENCE, ASSOCIATION FOR RESEARCH IN VISION AND, US, vol. 46, no. Suppl S, 5 May 2005 (2005-05-05), page 5381, XP009073501, ISSN: 0146-0404
- MIELER W ET AL: "Safety evaluation of second year treatment of age-related macular degeneration with pegaptanib sodium (Macugen): VEGF inhibition study in ocular neovascularization (VISION)", INVESTIGATIVE OPHTHALMOLOGY & VISUAL SCIENCE, ASSOCIATION FOR RESEARCH IN VISION AND OPHTHALMOLOGY, US, vol. 46, no. suppl, 1 January 2005 (2005-01-01), page 1380, XP009100873, ISSN: 0146-0404
- SORBERA L A ET AL: "PAZOPANIB HYDROCHLORIDE. ONCOLYTIC, ANGIOGENESIS INHIBITOR, VEGFR-2 TYROSINE KINASE INHIBITOR", DRUGS OF THE FUTURE, BARCELONA, ES, vol. 31, no. 7, July 2006 (2006-07), pages 585-589, XP008076811, ISSN: 0377-8282

## Description

### FIELD OF THE INVENTION

The present invention relates to methods of treating ocular neovascular disorders in a mammal. The methods comprise administering pyrimidine derivatives and pharmaceutical compositions containing the same.

### BACKGROUND OF THE INVENTION

Neovascularization, also called angiogenesis, is the process of forming new blood vessels. Neovascularization occurs during normal development, and also plays an important role in wound healing following injury to a tissue. However, neovascularization has also been implicated as an important cause of a number of pathological states including, for example, cancer, rheumatoid arthritis, atherosclerosis, psoriasis, and diseases of the eye.

Eye diseases associated with vascular leaking and/or neovascularization are responsible for the vast majority of visual morbidity and blindness in developed countries (Campochiaro (2004) Expert Opin. Biol. Ther. 4:1395-402*).* One example of such a disorder is diabetic retinopathy, a common complication in individuals with diabetes mellitus and the fifth leading cause of new blindness. The most important contributors to the development of diabetic retinopathy are hyperglycemia and hypoxemia that lead to increased vasopermeability, endothelial cell proliferation, and pathological neovascularization (Chorostowska-Wynimko et al. J. Physiol. Pharmacol. (2005) 56 Suppl 4:65-70). These vascular abnormalities result in fluid leakage in the macula, which can result in progressive vision loss.

Another eye disorder in which neovascularization plays a role is age-related macular degeneration (AMD), which is the major cause of severe visual loss in the elderly. The vision loss in AMD results from choroidal neovascularization (CNV). The neovascularization originates from choroidal blood vessels and grows through Bruch's membrane, usually at multiple sites, into the sub-retinal pigmented epithelial space and/or the retina (see, for example, Campochiaro et al. (1999) Mol. Vis. 5:34). Leakage and bleeding from these new blood vessels results in vision loss.

Eye disorders associated with ocular neovascularization are a major cause of vision loss and blindness. Accordingly, there remains a need for new methods of treating ocular neovascular disorders.

### SUMMARY OF THE INVENTION

The present invention is directed to new methods for treating ocular neovascular disorders. The methods comprise the step of administering pyrimidine derivatives, and pharmaceutical compositions containing the same.

In one aspect, the invention provides an oral pharmaceutical formulation comprising a compound of formula (I) 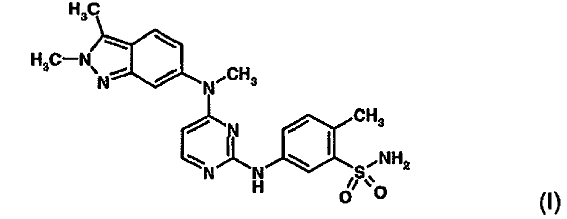 or salt or solvate thereof, for use in the treatment of ocular neovascular disorders.

>

The invention also encompasses the use of a compound of formula (I), or salt or solvate thereof for the manufacture of a medicament for oral administration useful in the treatment of ocular neovascular disorders.

Also provided is a compound of formula (I), or salt or solvate thereof for use in the treatment of ocular neovascular disorders by oral administration.

### BRIEF DESCRIPTION OF THE DRAWINGS

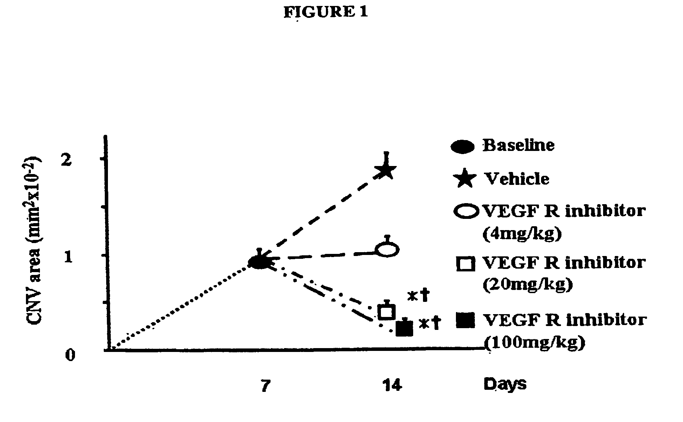

**Figure 1** shows the effect of the VEGF receptor inhibitor described in Example 1 in a regression model for choroidal neovascularization (CNV) in mice. In this regression model, CNV was induced in mice by laser bums on the posterior pole of the retina. Seven days after the laser-induced injury, the mice began a regime in which they were given either vehicle alone or 5-[[4-[(2,3-Dimethyl-2H-indazol-6-yl)methylamino]-2-pyrimidinyl]amino]-2-methylbenzenesulfonamide at the indicated doses. Seven days after this regime was initiated, the size of the CNV lesions was quantitated. The results are graphically summarized in Figure 1. See the Examples section for additional information.

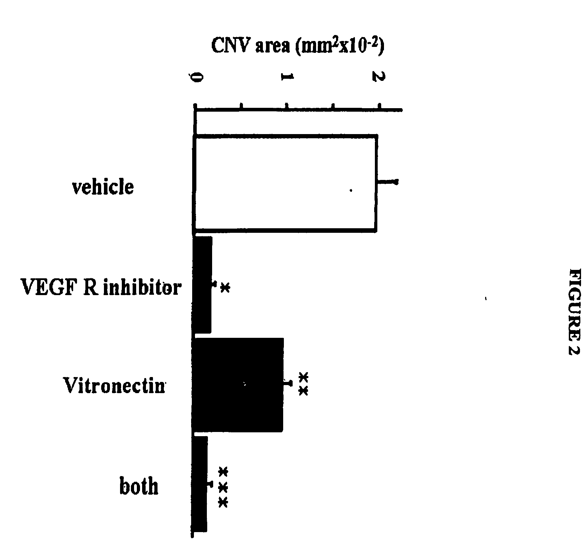

**Figure 2** shows the effect of pre-treatment with the VEGF receptor inhibitor described in Example 1, the vitronectin receptor antagonist described in Example 3, or a combination thereof on injury-induced CNV in mice in a prevention model for CNV. The results are graphically summarized in Figure 1. See the Examples section for additional information.

### DETAILED DESCRIPTION OF THE INVENTION

The present invention provides new methods of treating ocular neovascular disorders in mammals. The methods comprise the step of administering pyrimidine derivatives, and pharmaceutical compositions containing the same to a mammal. According to one aspect, the compound to be administered is pazopanib ((5-({4-[(2,3-dimethyl-2H-indazol-6-yl)(methyl)amino]pyrimidin-2-yl}amino)-2-methylbenzenesulfonamide) or a salt or solvate thereof. The present inventors have demonstrated that mice that are treated with pazopanib following a laser-induced injury to the retina show a decrease in the size of the resulting choroidal neovascular lesions when compared with untreated mice. In addition, the inventors have shown that mice treated with pazopanib, (S)-3-oxo-8-[3-(pyridin-2-ylamino)-1-propyloxy]-2-(2,2,2-trifluoroethyl)-2,3,4,5-tetrahydro-1H-2-benzazepine-4-acetic acid or a combination of these compounds prior to laser-induced injury to the retina show a decrease in size of the resulting choroidal neovascular lesions. Accordingly, the inventors have demonstrated that pazopanib, (S)-3-oxo-8-[3-(pyridin-2-ylamino)-1-propyloxy]-2-(2,2,2-trifluoroethyl)-2,3,4,5-tetrahydro-1H-2-benzazepine-4-acetic acid, and their derivatives, salts, and solvates are useful as therapeutic agents for treating disorders associated with neovascularization in the eye.

In one aspect, the invention provides an oral pharmaceutical formulation comprising a compound of formula (I): 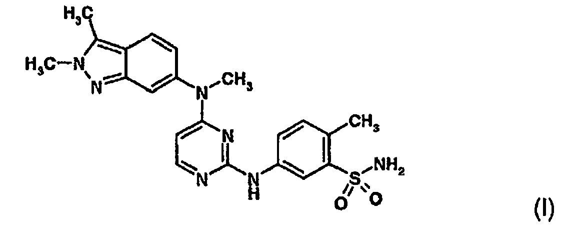 or salt or solvate thereof, for use in the treament of ocular neovascular disorders.

In a particular embodiment, the invention provides an oral pharmaceutical formulation for use in the treatment of ocular neovascular disorders comprising a compound of formula (I'):

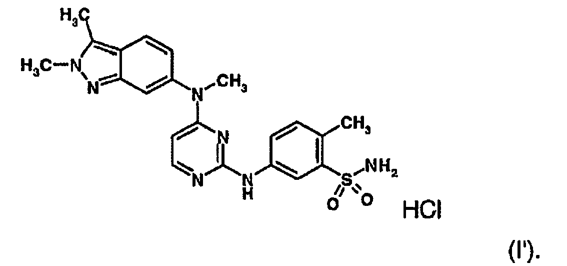

In another embodiment, the invention encompasses an oral pharmaceutical formulation for use in the treatment of ocular neovascular disorders comprising a compound of formula (I"):

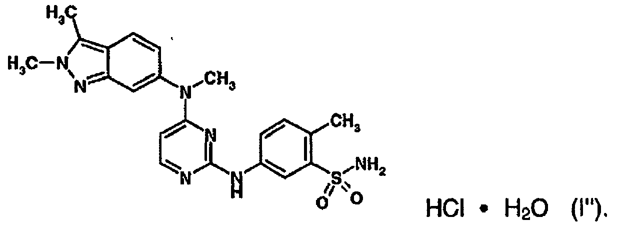

In another aspect, the invention encompasses the use of a compound of formula (I), or salt or solvate thereof for the preparation of a medicament useful in the treatment of ocular neovascular disorders by oral administration.

Also provided is a compound of formula (I), or a salt or solvate thereof for use in in the treatment of ocular neovascular disorders by oral administration.

In some embodiments of the invention, the ocular neovascular disorder is a choroidal neovascular disorder or a retinal neovascular disorder. In particular embodiments, the ocular neovascular disorder is selected from exudative age-related macular degeneration, angiod streaks, uveitis, and macular edema.

The term "ocular neovascular disorder" as used herein means a disorder in which new blood vessels are generated in the eye in a pathogenic manner. Ocular neovascular disorders that may be treated according to the methods of the invention include those characterized by vascular leakage. Ocular neovascular disorders may result in partial or full loss of vision. The neovascular disorders to be treated in the methods of the invention may occur in any part of the eye including, for example, the cornea, iris, retina, vitreous, and choroid.

The term "choroidal neovascular disorder" as used herein means a disorder characterized by an invasion of new blood vessels through Bruch's membrane, the innermost layer of the choroid.

The term "retinal neovascular disorder" as used herein refers to a disorder associated with the growth of new blood vessels originating from the retinal veins and extending along the vitreal surface of the retina.

Non-limiting examples of ocular vascular disorders that may be treated according to the methods of the invention include exudative age-related macular degeneration (AMD), angiod streaks, pathological myopia, ocular histoplasmosis syndrome, breaks in Bruch's membrane, macular edema (including diabetic macular edema), sarcoidosis and uveitis. Additional examples of disorders that may be treated by the disclosed methods include atrophic AMD, keratoconus, Sjogren's syndrome, myopia, ocular tumors, corneal graft rejection, corneal injury, neovascular glaucoma, corneal ulceration, corneal scarring, proliferative vitreoretinopathy, retinopathy of prematurity, retinal degeneration, chronic glaucoma, retinal detachment, and sickle cell retinopathy.

The invention provides methods for the treatment of ocular neovascular disorders. As used herein, "treatment" means any manner in which one or more symptoms associated with the disorder are beneficially altered. Accordingly, the term includes healing, prevention, or amelioration of a symptom or side effect of the disorder or a decrease in the rate of advancement of the disorder.

According to the invention, treatment of an ocular vascular disorder may be obtained by the administration of an effective amount of one or more therapeutic agents to the subject to be treated. As used herein, the term "effective amount" means the amount of a therapeutic agent that is sufficient to treat, prevent and/or ameliorate one or more symptoms of the disorder.

As used herein, the term "solvate" refers to a complex of variable stoichiometry formed by a solute (in this invention, compounds of formula (I), (II), (111), or a salt thereof) and a solvent. Such solvents for the purpose of the invention may not interfere with the biological activity of the solute. Examples of suitable solvents include, but are not limited to, water, methanol, ethanol and acetic acid. Preferably the solvent used is a pharmaceutically acceptable solvent. Examples of suitable pharmaceutically acceptable solvents include, without limitation, water, ethanol and acetic acid. In particular embodiments, the solvent used is water.

In one embodiment, the methods of preventing or treating ocular neovascular disorders disclosed herein include administering a compound of formula (I): 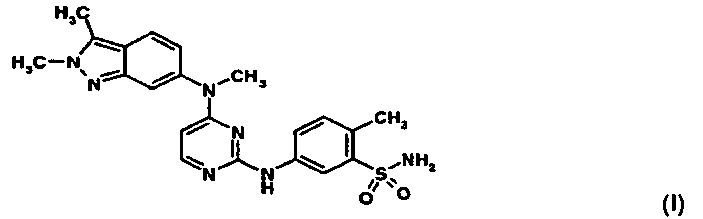 or a salt or solvate thereof.

In certain embodiment, the salt of the compound of formula (I) is a hydrochloride salt. In a particular embodiment, the salt of the compound of formula (I) is a monohydrochloride salt as illustrated by formula (I'). The monohydrochloride salt of the compound of formula (I) has the chemical name 5-[[4-[(2,3-Dimethyl-2H-indazol-6-yl)methylamino]-2-pyrimidinyl]amino]-2-methylbenzenesulfonamide monohydrochloride.

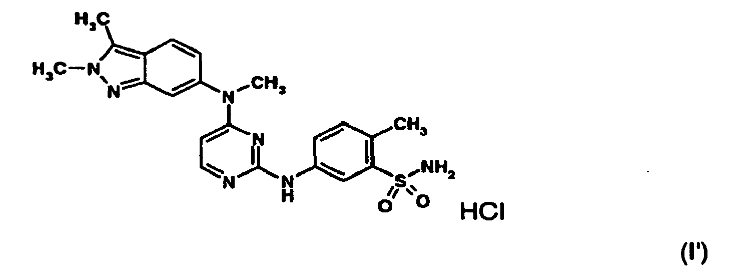

In another embodiment, the salt of the compound of formula (I) is a monohydrochloride monohydrate solvate of the compound of formula (I). The monohydrochloride monohydrate solvate of the compound of formula (I) has the chemical name 5-({4-[(2,3-dimethyl-2H-indazol-6-yl)methylamino]-2-pyrimidinyl)amino)-2-methylbenzenesulfonamide monohydrochloride monohydrate, as illustrated in formula (I").

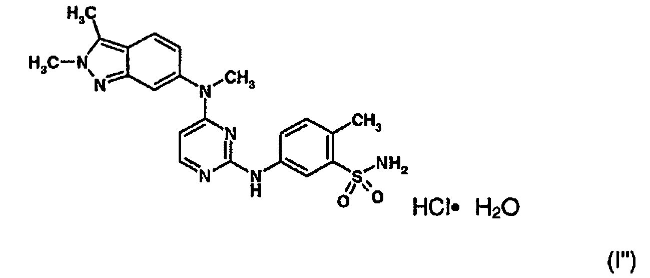

The free base, salts and solvates of the compound of formula (I) may be prepared, for example, according to the procedures of International Patent Application No. PCT/US01/49367 filed December 19, 2001, and published as WO 02/059110 on August 1, 2002, and International Patent Application No. PCT/US03/19211 filed June 17, 2003, and published as WO 03/106416 on December 24, 2003, or according the methods provided herein.

Typically, the salts of the present invention are pharmaceutically acceptable salts. Salts encompassed within the term "pharmaceutically acceptable salts" refer to non-toxic salts of the compounds of this invention. Salts of the compounds of the present invention may comprise acid addition salts derived from a nitrogen on a substituent in a compound of the present invention. Representative salts include the following salts: acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, laurate, malate, maleate, mandelate, mesylate, methylbromide, methylnitrate, methylsulfate, monopotassium maleate, mucate, napsylate, nitrate, N-methylglucamine, oxalate, pamoate (embonate), palmitate, pantothenate, phosphate/diphosphate, polygalacturonate, potassium, salicylate, sodium, stearate, subacetate, succinate, tannate, tartrate, teoclate, tosylate, triethiodide, trimethylammonium and valerate. Other salts, which are not pharmaceutically acceptable, may be useful in the preparation of compounds of this invention and these form a further aspect of the invention.

The compounds used in the methods of the invention may be administered alone, or they may be administered in a pharmaceutical composition. Accordingly, the invention further provides for the use of pharmaceutical compositions in the treatment methods of the present invention. The pharmaceutical compositions include a compound of formula (I), and salts or solvates thereof, and one or more pharmaceutically acceptable carriers, diluents, or excipients. The carrier(s), diluent(s) or excipient(s) must be acceptable in the sense of being compatible with the other ingredients of the formulation and not deleterious to the recipient thereof.

Pharmaceutical formulations may be presented in unit dose forms containing a predetermined amount of active ingredient per unit dose. Such a unit may contain, for example, 1µg to 1 g, such as 5µg to 500µg, 10µg-250µg, 0.5mg to 700mg, 2mg to 350mg, or 5mg to100 mg of a compound of formula (I), or salts or solvates thereof depending on the condition being treated, the route of administration and the age, weight and condition of the patient, or pharmaceutical formulations may be presented in unit dose forms containing a predetermined amount of active ingredient per unit dose. In certain embodiments, the unit dosage formulations are those containing a daily dose or sub-dose, as herein above recited, or an appropriate fraction thereof, of an active ingredient. Furthermore, such pharmaceutical formulations may be prepared by any of the methods well known in the pharmacy art.

The compound of formula (I), or salt or solvate thereof may be administered by the oral.

The methods of the present invention may also be employed in combination with other methods for the treatment of ocular neovascular disorders. In some embodiments, the methods of the invention encompass a combination therapy in which a compound of formula (I), or a salt or solvate thereof is administered in conjunction with one or more additional therapeutic agents for the treatment of neovascular disorders. Non-limiting examples of additional therapeutic agents that may be used in a combination therapy include pegaptanib, ranibizumab, PKC412, nepafenac, and integrin receptor antagonists (including vitronectin receptor agonists). See, for example, Takahashi et al. (2003) Invest. Ophthalmol. Vis. Sci. 44: 409-15, Campochiaro et al. (2004) Invest. Ophthalmol. Vis. Sci. 45:922-31, van Wijngaarden et al. (2005) JAMA 293:1509-13, U.S. Patent No. 6,825,188 to Callahan et al.*,* and U.S. Patent No. 6,881,736 to Manley et al.*;* each of which is herein incorporated by reference for their teachings regarding these compounds.

Where a combination therapy is employed, the therapeutic agents may be administered together or separately. The same means for administration may be used for more than one therapeutic agent of the combination therapy; alternatively, different therapeutic agents of the combination therapy may be administered by different means. When the therapeutic agents are administered separately, they may be administered simultaneously or sequentially in any order, both close and remote in time. The amounts of the compound of formula (I), and/or and the other pharmaceutically active agent or agents and the relative timings of administration will be selected in order to achieve the desired combined therapeutic effect.

Pharmaceutical formulations adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

For instance, for oral administration in the form of a tablet or capsule, the active drug component can be combined with an oral, non-toxic pharmaceutically acceptable inert carrier such as ethanol, glycerol, water and the like. Powders can be prepared by comminuting the compound to a suitable fine size and mixing with a similarly comminuted pharmaceutical carrier such as an edible carbohydrate, as, for example, starch or mannitol. Flavoring, preservative, dispersing and coloring agent can also be present.

Capsules can be made by preparing a powder mixture as described above, and filling formed gelatin sheaths. Glidants and lubricants such as colloidal silica, talc, magnesium stearate, calcium stearate or solid polyethylene glycol can be added to the powder mixture before the filling operation. A disintegrating or solubilizing agent such as agar-agar, calcium carbonate or sodium carbonate can also be added to improve the availability of the medicament when the capsule is ingested.

Moreover, when desired or necessary, suitable binders, lubricants, disintegrating agents and coloring agents can also be incorporated into the mixture. Suitable binders include starch, gelatin, natural sugars such as glucose or beta-lactose, com sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methyl cellulose, agar, bentonite, xanthan gum and the like. Tablets can be formulated, for example, by preparing a powder mixture, granulating or slugging, adding a lubricant and disintegrant and pressing into tablets. A powder mixture is prepared by mixing the compound, suitably comminuted, with a diluent or base as described above, and optionally, with a binder such as carboxymethylcellulose, an aliginate, gelatin, or polyvinyl pyrrolidone, a solution retardant such as paraffin, a resorption accelerator such as a quaternary salt and/or an absorption agent such as bentonite, kaolin or dicalcium phosphate. The powder mixture can be granulated by wetting with a binder such as syrup, starch paste, acadia mucilage or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc or mineral oil. The lubricated mixture is then compressed into tablets. The compounds of the present invention can also be combined with free flowing inert carrier and compressed into tablets directly without going through the granulating or slugging steps. A clear or opaque protective coating consisting of a sealing coat of shellac, a coating of sugar or polymeric material and a polish coating of wax can be provided. Dyestuffs can be added to these coatings to distinguish different unit dosages.

Oral fluids such as solution, syrups and elixirs can be prepared in dosage unit form so that a given quantity contains a predetermined amount of the compound. Syrups can be prepared by dissolving the compound in a suitably flavored aqueous solution, while elixirs can be prepared through the use of a non-toxic alcoholic vehicle. Suspensions can be formulated by dispersing the compound in a non-toxic vehicle. Solubilizers and emulsifiers such as ethoxylated isostearyl alcohols and polyoxy ethylene sorbitol ethers, preservatives, flavor additive such as peppermint oil or natural sweeteners or saccharin or other artificial sweeteners, and the like can also be added.

Where appropriate, dosage unit formulations for oral administration can be microencapsulated. The formulation can also be prepared to prolong or sustain the release as for example by coating or embedding particulate material in polymers, wax or the like.

The agents for use according to the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Agents for use according to the present invention may also be delivered by the use of monoclonal antibodies as individual carriers to which the compound molecules are coupled. The compounds may also be coupled with soluble polymers as targetable drug carriers. Such polymers can include polyvinylpyrrolidone, pyran copolymer, polyhydroxypropylmethacrylamide-phenol, polyhydroxyethylaspartamidephenol, or polyethyleneoxidepolylysine substituted with palmitoyl residues. Furthermore, the compounds may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates and cross-linked or amphipathic block copolymers of hydrogels.

Pharmaceutical formulations adapted for topical administration in the mouth include lozenges, pastilles and mouth washes.

It is understood by those skilled in the art that in addition to the ingredients particularly mentioned above, the formulations may include other agents conventional in the art having regard to the type of formulation in question. For example, those suitable for oral administration may include flavoring agents.

According to the methods of the invention, a specific compound of formula (I), is administered to a mammal. Typically, the amount of one of the administered agents of the present invention will depend upon a number of factors including, for example, the age and weight of the mammal, the precise condition requiring treatment, the severity of the condition, the nature of the formulation, and the route of administration. Ultimately, the amount will be at the discretion of the attendant physician or veterinarian.

Typically, the compound of formula (I), or salt or solvate thereof will be given in the range of 0.1 to 100 mg/kg body weight of recipient (mammal) per day and more usually in the range of 1 to 10 mg/kg body weight per day. In particular embodiments the compound is administered locally (for example, to the eye) and the total amount of a compound administered may be 1µg to 10 mg, such as 5µg to 500µg, or 10µg-250µg.

### EXAMPLES

As used herein the symbols and conventions used in these processes, schemes and examples are consistent with those used in the contemporary scientific literature, for example, the *Journal of the American Chemical Society* or the *Journal of Biological Chemistry.* Standard single-letter or three-letter abbreviations are generally used to designate amino acid residues, which are assumed to be in the L-configuration unless otherwise noted. Unless otherwise noted, all starting materials were obtained from commercial suppliers and used without further purification. Specifically, the following abbreviations may be used in the examples and throughout the specification:

| | |
|---|---|
| g (grams); | mg (milligrams); |
| L (liters); | mL (milliliters); |
| µL (microliters); | psi (pounds per square inch); |
| M (molar); | mM (millimolar); |
| N (Normal) | Kg (kilogram) |
| i. v. (intravenous); | Hz (Hertz); |
| MHz (megahertz); | mol (moles); |
| mmol (millimoles); | RT (room temperature); |
| min (minutes); | h (hours); |
| mp (melting point); | TLC (thin layer chromatography); |
| Tᵣ (retention time); | RP (reverse phase); |
| DCM (dichloromethane); | DCE (dichloroethane); |
| DMF (*N*,*N*-dimethylformamide); | HOAc (acetic acid); |
| TMSE (2-(trimethylsilyl)ethyl); | TMS (trimethylsilyl); |
| TIPS (triisopropylsilyl); | TBS (*t*-butyldimethylsilyl); |
| HPLC (high pressure liquid chromatography); | |
| THF (tetrahydrofuran); | DMSO (dimethylsulfoxide); |
| EtOAc (ethyl acetate); | DME (1,2-dimethoxyethane); |
| EDTA | ethylenediaminetetraacetic acid |
| FBS | fetal bovine serum |
| IMDM | Iscove's Modified Dulbecco's medium |
| PBS | phosphate buffered saline |
| RPMI | Roswell Park Memorial Institute |
| RIPA buffer | * |
| RT | room temperature |
| *150 mM NaCl, 50 mM Tris-HCl, pH 7.5, 0.25% (w/v) -deoxycholate, 1% NP-40, 5 mM sodium orthovanadate, 2 mM sodium fluoride, and a protease inhibitor cocktail. | |

Unless otherwise indicated, all temperatures are expressed in **°**C (degrees Centigrade). All reactions conducted under an inert atmosphere at room temperature unless otherwise noted.

The following examples describe the syntheses of intermediates particularly useful in the synthesis of compounds of formula (I) :

### Intermediate Example 1

### Preparation of 2,3-dimethyl-6-nitro-2H-indazole

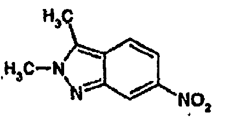

### Procedure 1:

To a stirred solution of 18.5 g (0.11 mol) of 3-methyl-6-nitro-*1H*-indazole in 350 ml acetone, at room temperature, was added 20 g (0.14 mol) of trimethyloxonium tetraflouroborate. After the solution was allowed to stir under argon for 3 hours, the solvent was removed under reduced pressure. To the resulting solid was added saturated aqueous NaHCO₃ (600 mL) and a 4:1 mixture of chloroform-isopropanol (200 ml), the mixture was agitated and the layers were separated. The aqueous phase was washed with additional chloroform: isopropanol (4 x 200 mL) and the combined organic phase was dried (Na₂SO₄). Filtration and removal of solvent gave a tan solid. The solid was washed with ether (200 mL) to afford 2,3-dimethyl-6-nitro-*2H*-indazole as a yellow solid (15.85 g, 73 %). ¹H NMR (300 MHz, DMSO-d₆) δ 8.51 (s, 1H), 7.94 (d, J = 9.1 Hz, 1H), 7.73 (d, J = 8.9 Hz, 1H), 4.14 (s, 3H), 2.67 (s, 3H). MS (ES+, m/z) 192 (M+H).

### Procedure 2:

Trimethyl orthoformate (11 mmol, 1.17 g) was added over a 2 min period to a solution of boron trifluoride etherate (12.5 mmol, 1.77 g in methylene chloride (2.0 mL) which had been cooled to -30 **°**C**.** The mixture was warmed to 0 **°**C for 15 min and was then cooled to -70 **°**C**.** The nitro indazole (10 mmol, 1.77 g) was slurried in methylene chloride (30 mL) and was added all at once to the cooled mixture. The mixture was stirred at -70 **°**C for 15 min and at ambient temperature for 17 h. After 17 h the mixture was red and heterogeneous. The reaction mixture was quenched with saturated sodium bicarbonate solution (20 mL) and the organic layer separated. The aqueous layer was extracted with methylene chloride (30 mL). The methylene chloride layers were combined and extracted with water (30 mL). The methylene chloride layer was distilled under reduced pressure until - 10 mL remained. Propanol (10 mL) was added and the remainder of the methylene chloride removed under reduced pressure, resulting in a yellow slurry. The product was isolated by filtration to give 2,3-dimethyl-6-nitro-*2H*-indazole (65 %, 7mmol, 1.25 g) as a light yellow powder. ¹H NMR (300 MHz, DMSO-d₆) δ 8.51 (s, 1H), 7.94 (d, J= 9.1 Hz, 1H), 7.73 (d, J= 8.9 Hz, 1H), 4.14 (s, 3H), 2.67 (s, 3H). MS (ES+, m/z) 192 (M+H).

### Procedure 3:

In a 25 ml round bottom flask 3-methyl-6-nitroindazole (7.27 mmol, 1.28 g) was dissolved with stirring in DMSO (4.0 mL) and was treated with concentrated sulfuric acid (7.27 mmol, 0.73 g) to yield a thick slurry. The slurry was treated with dimethyl sulfate (21.1 mmol, 2.66 g). The mixture was heated under nitrogen at 50 **°**C for 72 h. After 72 h a thick yellow slurry was obtained. The slurry was cooled and was slowly treated with saturated sodium bicarbonate solution (10 mL). The mixture was extracted with methylene chloride (2 x 20 mL). The methylene chloride layers were combined and back extracted with water (20 mL). The methylene chloride layer was treated with propanol (10 mL) and the methylene chloride was removed by distillation under reduced pressure. The solid was isolated by filtration and the yellow solid washed with heptane (5 mL) and air-dried. The 2,3-dimethyl-6-nitro-*2H*+indazole product (70%, 0.97 g) was obtained as a light yellow solid. ¹H NMR (300 MHz, DMSO-d₆) δ 8.51 (s, 1H), 7.94 (d, J = 9.1 Hz, 1H), 7.73 (d, J = 8.9 Hz, 1H), 4.14 (s, 3H), 2.67 (s, 3H). MS (ES+, m/z) 192 (M+H).

### Procedure 4:

Into a 250 mL 3-necked round bottom flask was placed 3-methyl-6-nitro-1H-indazole sulfuric acid salt (5.0 g, 18.2 mmol) and methylene chloride (25 mL). The mixture was stirred at 25 **°**C and was treated with DMSO (5 mL). Dimethyl sulfate (6.7 g, 5.0 mL, 53.0 mmol) was added via syringe and the reaction was heated at reflux in a 70 **°**C bath. After 7 h HPLC analysis showed 9% starting material. At this point heating was stopped and the workup begun. Saturated sodium bicarbonate solution (35 mL) was added to the reaction mixture at RT. The layers were allowed to separate and the aqueous layer was extracted with methylene chloride (25 mL). The methylene chloride layers were combined and washed with water (2 x 25 mL). The methylene chloride layer was distilled under reduced pressure until half the volume was removed. Propanol (25 mL) was added and distillation under reduced pressure was continued until all the methylene chloride had been removed. This yielded a yellow slurry, which was allowed to stir at 25 **°**C for 1 h. The product was isolated via filtration and the resulting yellow solid was washed with heptane (10 mL). This yielded 2,3-dimethyl-6-nitro-2H-indazole (70%, 2.43 g) as a yellow solid. ¹H NMR (300 MHz, DMSO-d₆) δ 8.51 (s, 1H), 7.94 (d, *J* = 9.1 Hz, 1H), 7.73 (d, *J* = 8.9 Hz, 1H), 4.14 (s, 3H), 2.67 (s, 3H). MS (ES+, m/z) 192 (M+H).

### Intermediate Example 2

### Preparation of 2,3-dimethyl-6-amino-2H-indazole:

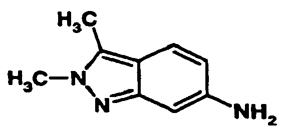

### Procedure 1:

To a stirred solution of 2,3-dimethyl-6-nitro-*2H*-indazole (1.13 g) in 2-methoxyethyl ether (12 ml), at 0 **°**C, was added a solution of 4.48 g of tin(II) chloride in 8.9 ml of concentrated HCl dropwise over 5 min. After the addition was complete, the ice bath was removed and the solution was allowed to stir for an additional 30 min. Approximately 40 ml of diethyl ether was added to reaction, resulting in precipitate formation. The resulting precipitate was isolated by filtration and washed with diethyl ether, and afforded a yellow solid (1.1 g, 95 %), the HCl salt 2,3-dimethyl-*2H* indazol-6-amine. ¹H NMR (300 MHz, DMSO-d₆) δ 7.77 (d, J= 8.9 Hz, 1H), 7.18 (s, 1H), 7.88 (m, 1H), 4.04 (s, 3H), 2.61 (s, 3H). MS (ES+, m/z) 162 (M+H).

### Procedure 2:

A 2-L 3-necked round bottom flask was fitted with nitrogen inlet and outlet and with mechanical stirring. A moderate nitrogen flow was initiated and the reactor was charged with 10 % Pd/C (50% water wet, 6.0 g). Stirring was initiated and the reactor was charged with methanol (750 mL) and the product of Intermediate Example 1 (50 g). Ammonium formate (82.54 g) was dissolved in water (120 mL). The water solution of ammonium formate was added to the reaction solution at an addition rate, which kept the reaction temperature at or between 25 and 30°C. The reaction was allowed to proceed at 25**°**C. After 6 h the reaction was judged to be finished based on HPLC analysis. The mixture was filtered and the catalyst washed with methanol (50 mL). The methanol layers were combined and the solvent removed under reduced pressure. The residue was dissolved in water (200 mL) and was extracted with methylene chloride (3 x 250 mL). The methylene chloride layers were combined and solvent removed under vacuum to remove approximately half the solvent. Heptane (400 mL) was added and the vacuum distillation continued until approximately 300 mL reaction product slurry remained. The product was isolated by filtration and dried under vacuum at 50**°**C for 4 h. to yield 2,3-dimethyl-6-amino-2H-indazole as the free base. (40.76 g, 96.7 %). ¹H NMR (300 MHz, DMSO-d₆) δ 7.31 (d, J= 8.9 Hz, 1H), 6.45 (d, J= 8.9 Hz, 1H), 6.38 (s, 1H), 4.95 (s, br, 2H), 3.85 (s, 3H), 2.44 (s, 3H) MS (ES+, m/z) 162 (M+H).

### Intermediate Example 3

### Preparation of N-(2-chloropyrimidin-4-yl)-2,3-dimethyl-2H-indazol-6-amine

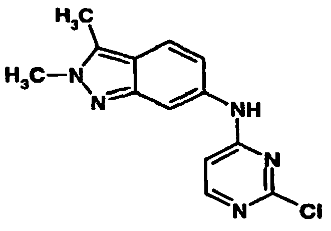

### Procedure 1

To a stirred solution of the product of Intermediate Example 2 (2.97 g, .015 mol) and NaHCO₃ (5.05 g, .06 mol) in THF (15 mL) and ethanol (60 mL) was added 2,4-dichloropyrimidine (6.70 g, .045 mol) at rt. After the reaction was stirred for four hours at 85 **°**C, the suspension was cooled to rt., filtered and washed thoroughly with ethyl acetate. The filtrate was concentrated under reduced pressure, and the resulting solid was triturated with ethyl acetate to yield *N*-(2-chloropyrimidin-4-yl)-2,3-dimethyl-*2H-*indazol-6-amine (89 %, 3.84 g). ¹H NMR (400 MHz, DMSO-d₆) δ 7.28 (d, *J* = 9.0 Hz, 1H), 6.42 (d, J = 8.8 Hz, 1H), 6.37 (s, 1H), 5.18 (br s, 1H), 3.84 (s, 3H), 2.43 (s, 3H). MS (ES+, m/z) 274 (M+H).

### Procedure 2

To a 1-L 3-necked flask equipped with air-driven mechanical stirrer, thermometer, and nitrogen inlet/outlet was charged a solution of the product of Intermediate Example 2 (32.89 g, 0.204 mol, 1.0 equiv) in 425 mL (13 volumes) of EtOH/THF (4/1), sodium bicarbonate (51.42 g, 0.612 mol, 3.0 equiv) and then 2,4-dichloropyrimidine (45.59 g, 0.306 mol, 1.5 equiv). The flask contents were heated to 75**°**C and held at 74―76 **°**C for 6 ―7 hrs. The progress of the reaction was checked by HPLC (the product of Intermediate Example 2 < 2%). The reaction contents were cooled to 20 ― 25 **°**C over 30 min, and kept at 20 ― 25 **°**C for 30 min. Then the reaction contents were further cooled to 10 - 12**°**C over 30 min, and kept at that temperature for an additional 10 min. The contents were filtered and filter cake washed with EtOAc (2 x 100 mL, 3.0 volumes), and deionized water (514 mL, 15.6 volumes). The filter cake was then dried in a vacuum oven at 35**°**C overnight to afford the desired product 44.75 g as a white solid (80.1 %). ¹H NMR (400 MHz, DMSO-d₆) δ 7.28 (d, J= 9.0 Hz, 1H), 6.42 (d, J = 8.8 Hz, 1H), 6.37 (s, 1H), 5.18 (br s, 1H), 3.84 (s, 3H), 2.43 (s, 3H). MS (ES+, m/z) 274 (M+H).

### Procedure 3

To a 2 L jacketed reactor was charged with IMS (1000 mL), the product of Intermediate Example 2 (100 g, 0.620 mol, 1 equiv), Sodium Hydrogen Carbonate (107g, 1.27 mol, 2.05 equiv), and 2,4-dichloropyrimidine (101 g, 0.682 mol, 1.1 equiv). The solution was stirred and heated to reflux with a jacket temperature of 85 °C for 8 hours. The resulting slurry was then cooled to 50 **°**C, and water (500 mL) was added to maintain the temperature between 40 and 50**°**C. The reaction was then stirred at an internal temperature of 50**°**C for one hour, and then cooled to 20**°**C. The solid product was collected by filtration, washed with water (750 mL X 2), and followed by with EtOAc (450 mL X 1). After drying at overnight, under vacuum at 60 **°**C afforded 135 g (80%) of *N*-(2-chloropyrimidin-4-yl)-2,3-dimethyl-2*H*-indazol)-6-amine.

### Intermediate Example 4

### Preparation of N-(2-chloropyrimidin-4-yl)-N,2,3-trimethyl-2H-indazol-6-amine

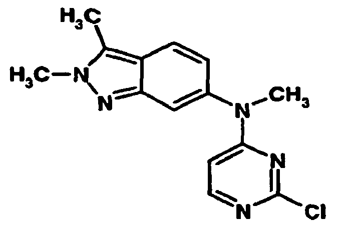

### Procedure 1

To a stirred solution of the product of Intermediate Example 3 (7.37 g) in DMF (50 ml) was added Cs₂CO₃ (7.44 g, 2 eqv.) and iodomethane (1.84 ml, 1.1 eqv.) at room temperature. The mixture was stirred at rt overnight. The reaction mixture was then poured into an ice-water bath, and the precipitate was collected via filtration and washed with water. The precipitate was air-dried to afford *N*-(2-chloropyrimidin-4-yl)-*N*,2,3-trimethyl-2*Hi-*indazol-6-amine as an off-white solid (6.43 g, 83%). ¹H NMR (400 MHz, DMSO-d₆) δ 7.94 (d, J = 6.0 Hz, 1H), 7.80 (d, J = 7.0 Hz, 1H), 7.50 (d, J = 1.0 Hz, 1H), 6.88 (m, 1H), 6.24 (d, J = 6.2 Hz, 1H), 4.06 (s, 3H), 3.42 (s, 3H), 2.62 (s, 3H). MS (ES+, m/z) 288 (M+H).

### Procedure 2

A 3L 3-necked flask equipped with air-driven mechanical stirrer, thermometer, addition funnel and nitrogen inlet/outlet was charged with DMF (272 mL, 5 volumes) and the product of Intermediate Example 3 (54.4 g, 0.20 mol, 1.0 equiv) with stirring. The reaction mixture was further charged with cesium carbonate (194.5 g, 0.60 mol, 3.0 equiv) while maintaining the reaction temperature between 20 - 25**°**C. The reaction mixture was stirred at 20 - 25°C for 10 minutes. Iodomethane (45.1 g, 0.32 mol, 1.6 equiv) was charged over ~ 10 minutes while maintaining the temperature 20 - 30**°**C. The reaction mixture was stirred at 20 - 30**°**C (Typically, the reaction is complete in 1 - 2 hours). Deionized H₂O (925 mL, 17 volumes) was added over - 30 minutes while maintaining the temperature at 25 - 40°C. The reaction mixture was stirred at 20 - 25**°**C for 40 minutes. The product was isolated by filtration and then the filter cake washed with H₂O / DMF (6 : 1, 252 mL, 4.6 volumes). The wet cake was dried under vacuum at 40 - 45**°**C and *N-*(2-chloropyrimidin-4-yl)-*N*,2,3-trimethyt-2*H*-indazot-6-amine (51.7 g, 90.4%) was isolated as a yellow solid.¹H NMR (400 MHz, DMSO-d₆) δ 7.94 (d, J = 6.0 Hz, 1H), 7.80 (d, J = 7.0 Hz, 1H), 7.50 (d, J = 1.0 Hz, 1H), 6.88 (m, 1H), 6.24 (d, J = 6.2 Hz, 1H), 4.06 (s, 3H), 3.42 (s, 3H), 2.62 (s, 3H). MS (ES+, m/z) 288 (M+H).

### Procedure 3

To a 2 L jacketed reactor was charged with DMF (383 mL), dimethyl carbonate (192 mL), the product of Intermediate Example 3 (115 g, 0.420 mol, 1 equiv) and Potassium Carbonate (174 g, 1.26 mol, 3 equiv). The suspension was stirred and heated to reflux with a jacket temperature of 135**°**C for 6 hours. The resulting slurry was then cooled to 60**°**C, and water (1150 mL) was added slowly maintaining the reaction temperature between 50 and 65 **°**C. The reaction was then cooled down to 20**°**C and stirred at an internal temperature of 20**°**C for two hours, and then cooled to 10°C and held overnight after which it was filtered. The solid was washed with water (230 mL X 2) at room temperature, and rinsed with the mixture IMS:Water (1:1) (230 mL X 1). After drying at overnight, under vacuum at 60**°**C afforded 101 g (83%) of N-(2-chloropyrimidin-4-yl)-N,2,3-trimethyl-2H-indazol-6-amine.

### Intermediate Example 5

### Preparation of 5-amino-2-methylbenzenesulfonamide

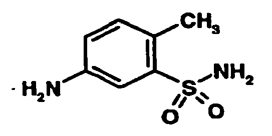

### Procedure 1

To a stirred solution of 2-methyl-5-nitrobenzenesulfonamide (4.6 g, 0.021 mol) in 2-methoxyethyl ether (43 mL), at 0^{o}C, was added a solution of 16.1 g of tin(II) chloride in 32 mL of concentrated HCl dropwise over 15 min. After the addition was complete, the ice bath was removed and the solution was allowed to stir for an additional 30 min. Approximately 130 mL of diethyl ether was added to reaction. The mixture was stirred vigorously for 1 h. The mixture was basified with a solution of NaOH and NaHCO₃, and extracted with ethyl acetate (x 3). The combined ethyl acetate layers were dried over anhydrous MgSO₄, filtered and concentrated to give crude product. Trituation of the crude product with methanol provided 2.4 g of pure 5-amino-2-methylbenzenesulfonamide as light brown solid. ¹H NMR (300 MHz, DMSO-d₆) δ 7.11-7.10 (m, 3H), 6.95 (d, J = 8.1 Hz, 1H), 6.60 (dd, J = 8.1 & 2.4 Hz, 1H), 5.24 (s, 2H), 2.36 (s, 3H). MS (ES+, m/z) 187 (M+H).

### Intermediate Example 6

### Preparation of 4-[(methylsulfonyl)methyl]aniline

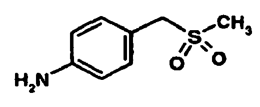

### Procedure 1

Combine 4-nitrobenzyl bromide (40 g, 0.185 mol) and sodium methanesulphinic acid (19.5 g, 1 eqv.) in ethanol (460 mL, -0.4M). The mixture was stirred and heated to 80 **°**C under reflux. After 3 hr the reaction mixture was cooled to rt and filtered to collected off-white solid. The solid was washed with EtOH twice and air-dried to provide 37 g of methyl 4-nitrobenzyl sulfone.¹H NMR (300 MHz, DMSO-d₆) δ 8.27 (d, J = 8.6 Hz, 2H), 7.69 (d, J = 8.6 Hz, 2H), 4.71 (s, 2H), 2.96 (s, 3H). MS (ES+, m/z) 216 (M+H).

Combined methyl 4-nitrobenzyl sulfone (9.5 g, 0.044 mol) and 10% Pd/C (0.95 g, 0.1 w/w) in ethyl acetate (220 mL, ~0.2M). The mixture was placed under Parr shaker with 40 psi of hydrogen. After ~3 hr, the reaction mixture was poured into 50% of MeOH/EtOAc (400 mL) and stirred vigorously for 30 min. The mixture was filtered through a pad of celite and silica gel. The black material on top of the pad was removed and placed into 80% MeOH/EtOAc (200 mL) and stirred vigorously for 30 min. The mixture was again filtered through a pad of celite and silica gel. The process is repeated a couple times. Combined all filtrates. Evaporated and dried. Trituation with EtOAc provided pure 4-[(methylsulfonyl)methyl]aniline. ¹H NMR (300 MHz, DMSO-d₆) δ 7.03 (d, J = 8.4 Hz, 2H), 6.54 (d, J = 8.6 Hz, 2H), 5.20 (s, 2H), 4.20 (s, 2H), 2.79 (s, 3H). MS (ES+, m/z) 186 (M+H).

### Procedure 2

Charge a round bottom flask (1.0 L), equipped with magnetic stir bar and reflux condenser, with 4-nitrobenzyl bromide (40 g, 0.185 mol, 1.0 eq.), sodium methanesulphinic acid (21.7 g, 0.213 mol, 1.15 eq.) and ethanol (400 mL, 200 proof, 10 vol.). Stir and heat the mixture to 80 **°**C under reflux for 2 hours. Check the progress of the reaction by fast-HPLC (reaction is deemed complete when HPLC indicates 4-nitrobenzyl bromide < 0.5%). Cool the mixture to room temperature. Filter and wash the cake with ethanol (40 mL). The wet cake (15 g, 46.2 mmol) was used for next step hydrogenation with out further dry.
Charge a 500 mL of hydrogenation flask with above wet cake methyl 4-nitrobenzyl sulfone (15 g, 46.2 mmol, used "as is"), 10% Pd/C (0.1 g, 1% w/w) and ethanol (120 mL, 200 proof) and water (40 mL). Swap the atmosphere of reactor with hydrogen (3 times). Shake the reactor under H₂ (65 psi) at room temperature for 30 minutes and at 50 **°**C for two hour. Check the progress of the reaction by HPLC (reaction is deemed complete when HPLC indicates methyl 4-nitrobenzyl sulfone < 0.2 %). Heat the mixture to 80 **°**C. Filter the hot solution through a pad of celite (2.0 g) and rinse the pad with EtOH (10 mL). Transfer the filtrate into the crystallizing a round bottom flask (500 mL). Distil the slurry under house vacuum at 60 °C until a volume of 60 mL is left. Cool the slurry to 0 **°**C over for one hour. Isolate the crystals by vacuum filtration and wash the vessel and crystals with ethanol (10 mL). Dry the product under house vacuum at 50 **°**C to constant weight. Obtained off-white solid (7.3 g). The yield is 85% for combined two steps with 99% purity of product by HPLC.

### Intermediate Example 7

### Preparation of 4-[(isopropylsu/fonyl)methyl]phenylamine

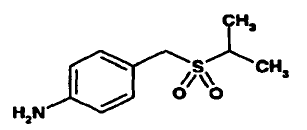

To a solution of 1-(bromomethyl)-4-nitrobenzene (3.0 g, 17.4 mmol) in ethanol (50 mL) was added sodium-2-thiopropoylate (2.7 g, 17.4 mmol). After 12h the solvent was removed under reduced pressure, the remaining residue was diluted with EtOAc and filtered to remove the residual salts. The solvent was dried over MgSO₄ and removed under reduced pressure and the product was carried forward without further purification. Next the sulfide was diluted with CH₂Cl₂ (50 mL) and m-chloroperoxybenzoic acid (~70%) (6.6 g, 38.4 mmol) was added in portions. The reaction was judged to be complete by tic and the solvent was removed under reduced pressure. The remaining residue was diluted with EtOAc and washed with 1 M NaOH (2 x 100 mL). The solvent was dried over MgSO₄ and removed under reduced pressure and the product was carried forward without further purification. Next the residue was diluted with glyme (8.0 mL) and a solution of SnCl₂ (13.8 g, 69 mmol) in HCl (8.0 mL) was added dropwise. The solution was allowed to stir for 2h, and the reduction was judged to be complete by tic. The reaction mixture was diluted with Et₂O, which resulted in the precipitation of the product as the HCl salt. The solids were collected and washed with Et₂O (2 x 100 mL), to afford pure aniline (-2.4 g, 65%). ¹H NMR (300 MHz, d₆DMSO+NaHCO₃) δ 7.37 (d, J = 8.4 Hz, 2H), 7.21 (d, J = 8.4 Hz, 2H), 4.41 (s, 2H), 3.18-3.09 (m, 1H), 1.21 (d, J= 6.9 Hz, 6H).

### Intermediate Example 8

### Preparation of 4-[2-(methylsulfonyl)ethyl]aniline

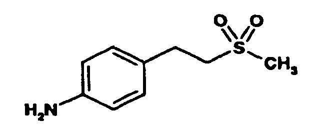

To a solution of 1-(bromoethyl)-4-nitrobenzene (3.0 g, 13.0 mmol) in ethanol (70 mL) was added Sodium thiomethoxide (1.0 g, 14.0 mmol). After 12h the solvent was removed under reduced pressure, the remaining residue was diluted with EtOAc and filtered to remove the residual salts. The solvent was dried over MgSO4 and removed under reduced pressure and the product was carried forward without further purification. Next the sulfide was diluted with CH₂Cl₂ (100 mL) and m-chloroperoxybenzoic acid (~70%) (8.2 g, 48.8 mmol) was added in portions. The reaction was judged to be complete by tlc and the solvent was removed under reduced pressure. The remaining residue was diluted with EtOAc and washed with 1 M NaOH (2 x 100 mL). The solvent was dried over MgSO₄ and removed under reduced pressure and the product was carried forward without further purification. Next the residue was added to a slurry of Palladium on Carbon (10 mol %) in EtOAc (50 mL) in a Parr shaker vessel. The reaction was then place under 40 atm of Hydrogen gas. The solution was allowed to shake for 2h, and the reduction was judged to be complete by tlc. The reaction mixture was filtered over a pad of celite and washed with EtOAc and the solvent was removed under reduced pressure to afford a crude solid. The mixture was recrystallized in hot EtOAc to afford the pure aniline (~1.8 g, 69%). ¹H NMR (300 MHz, d₆DMSO+NaHCO₃) δ 6.93 (d, J = 8.2 Hz, 2H), 6.87 (d, J = 8.2 Hz, 2H), 5.09 (bs, 2H), 3.31-3.26 (m, 2H), 2.92 (s, 3H), 2.84-2.79 (m, 2H).

### Intermediate Example 9

### Preparation of 4-[1-(methylsulfonyl)ethyl]aniline

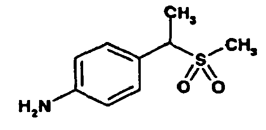

To a solution of 4-nitrophenylcarbonol (3.0 g, 17.9 mmol) and triethylamine (3.5 mL, 21.0 mmol) in CH₂Cl₂ (100mL) was added methanesulfonylchloride (1.7 mL, 21.0 mmol) dropwise. The reaction was judged to be complete by tlc after 1 h and was quenched with saturated aqueous NaHCO3. The reaction mixture was diluted with EtOAc and the organic layer separated, dried over MgSO₄ and the solvent was removed under reduced pressure. The resulting residue was dissolved in ethanol (100 mL) and Sodium thiomethoxide (1.5 g, 21.0 mmol) was added in portions. After 12h the solvent was removed under reduced pressure, the remaining residue was diluted with EtOAc and filtered to remove the residual salts. The solvent was dried over MgSO₄ and removed under reduced pressure and the product was carried forward without further purification. Next the sulfide was diluted with CH₂Cl₂ (100 mL) and m-chloroperoxybenzoic acid (~70%) (10.8 g, 62 mmol) was added in portions. The reaction was judged to be complete by tlc and the solvent was removed under reduced pressure. The remaining residue was diluted with EtOAc and washed with 1 M NaOH (2 x 100 mL). The solvent was dried over MgSO₄ and removed under reduced pressure and the product was carried forward without further purification. Next the residue was added to a slurry of Palladium on Carbon (10 mol %) in EtOAc (50 mL) in a Parr shaker vessel. The reaction was then place under 40 atm of Hydrogen gas. The solution was allowed to shake for 2h, and the reduction was judged to be complete by tlc. The reaction mixture was filtered over a pad of celite and washed with EtOAc and the solvent was removed under reduced pressure to afford a crude solid. The mixture was recrystallized in hot EtOAc to afford the pure aniline (~2.0 g, 57%). ¹H NMR (300 MHz, d₆DMSO+NaHCO₃) δ 7.06 (d, J= 8.5 Hz, 2H), 6.53 (d, J= 8.5 Hz, 2H), 5.21 (s, 2H), 4.23 (q, J= 7.1 Hz, 1H), 2.70 (s, 3H), 1.21 (d, J= 7.1 Hz, 3H).

### Intermediate Example 10

### Preparation of 4-[1-methyl-1-(methylsulfonyl)ethyl]aniline

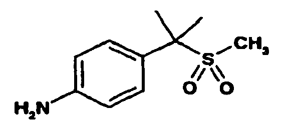

To a stirred solution of t-butoxide (5.76g, 0.051 mol) in THF was added methyl 4-nitrobenzyl sulfone (5 g, 0.023 mol) followed by iodomethane (2.89 ml, 0.046 mol). The mixture was stirred at rt for 1 hr. Additional t-butoxide (2.9 g) and iodomethane (0.5 ml) were added. The mixture was stirred at rt for additional 1 hr. The mixture was diluted with EtOAc and acidified with 6N HCl. The mixture was extracted with ethyl acetate (x 3). The combined ethyl acetate layers were dried over anhydrous MgSO4, filtered and evaporated. The solid was trituated with ethanol to give pure 1-[1-methyl-1-(methylsulfonyl)ethyl]-4-nitrobenzene.

To a stirred solution of 1-[1-methyl-1-(methylsulfonyl)ethyl]-4-nitrobenzene (3.32 g, 0.014 mol) in 2-methoxyethyl ether (70 mL), at 0ºC, was added a solution of 10.35 g of tin(II) chloride in 20.5 mL of concentrated HCl dropwise over 15 min. After the addition was complete, the ice bath was removed and the solution was allowed to stir for an additional 30 min. Approximately 70 mL of diethyl ether was added to reaction. The mixture was stirred vigorously for 1 h. Precipitate was formed and was collected via filtration. The solid was dissolved in CH₂Cl₂ and washed with 1 N NaOH. The mixture was extracted with CH₂Cl₂ (x 3). The combined CH₂Cl₂ layers were dried over anhydrous MgSO₄, filtered and evaporated to give 4-[1-methyl-1-(methylsulfonyl)ethyl]aniline as an off white solid. ¹H NMR (300 MHz, DMSO-d₆) δ 7.21 (d, J = 8.6 Hz, 2H), 6.55 (d, J = 8.6 Hz, 2H), 5.23 (s, 2H), 2.58 (s, 3H), 1.64 (s, 6H).

### Example 1: Preparation of pazopanib (5-({4-[(2,3-dimethyl-2H-indazol-6-yl)(methyl)amino]pyrimidin-2-yl}amino)-2-methylbenzenesulfonamide) and salts and solvates thereof

### Example 1 a

### Preparation of 5-({4-[(2,3-dimethyl-2H-indazol-6-yl)(methyl)amino]pyrimidin-2-yl}amino)-2-methylbenzenesulfonamide

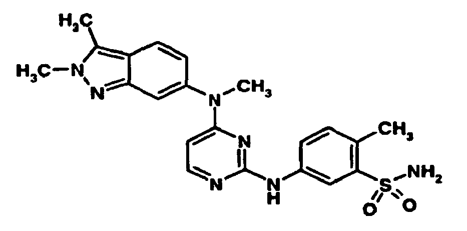

### Procedure 1

To a solution of Intermediate Example 4 (200 mg, 0.695 mmol) and 5-amino-2-methylbenzenesulfonamide (129.4 mg, 0.695 mmol) in isopropanol (6 ml) was added 4 drops of cone. HCl. The mixture was heated to reflux overnight. The mixture was cooled to rt and diluted with ether (6 ml). Precipitate was collected via filtration and washed with ether. The hydrochloride salt of 5-({4-[(2,3-dimethyl-*2H*-indazol-6-yl)(methyl)amino]-pyrimidin-2-yl}amino)-2-methylbenzenesulfonamide was isolated as an off-white solid. ¹H NMR (400 MHz, d₆DMSO+NaHCO₃) δ 9.50 (br s, 1H), 8.55 (br s, 1H), 7.81 (d, J = 6.2 Hz, 1H), 7.75 (d, J = 8.7 Hz, 1H), 7.69 (m, 1H), 7.43 (s, 1H), 7.23 (s, 2H), 7.15 (d, J = 8.4 Hz, 1H), 6.86 (m, 1H), 5.74 (d, J = 6.1 Hz, 1H), 4.04 (s, 3H), 3.48 (s, 3H), 2.61 (s, 3H), 2.48 (s, 3H). MS (ES+, m/z) 438 (M+H).

### Procedure 2

A 250-mL 3-necked flask equipped with a magnetic stir bar, thermometer, reflux condenser, and nitrogen inlet/outlet was charged with ethanol (60 mL, 10 volumes), the product of Intermediate Example 4 (6.00 g, 20.85 mmol, 1.0 equiv) and 5-amino-2-methylbenzenesulfonamide (4.00 g, 21.48 mmol, 1.03 equiv) with stirring. The reaction mixture was heated to 70 **°**C. After stirring the reaction mixture at 68 ― 72 **°**C for 3 hrs, 4M HCl in dioxane (0.11 mL, 0.44 mmol, 0.02 equiv) was charged over ca. 2 min. The reaction mixture was stirred at 68 - 72 **°**C until < 1.5% by area of the starting product of Intermediate Example 4 was remaining by HPLC analysis (Typically, this reaction is complete in > 8 hrs). The reaction mixture was cooled to 20 **°**C over ca. 30 min and stirred at 20 - 22 **°**C for 40 min. The product was then isolated by filtration and the filter cake washed with ethanol (20 mL, 3.3 volumes). The wet cake was dried under vacuum at 45 - 50 **°**C. The monohydrochloride salt of 5-({4-[(2,3-dimethyl-2*H*-indazol-6-yl)(methyl)amino]-pyrimidin-2-yl}amino)-2-methylbenzenesulfonamide (9.52 g, 96.4%) was isolated as a white solid. ¹H NMR (400 MHz, d₆DMSO+NaHCO₃) δ 9.50 (br s, 1H), 8.55 (br s, 1H), 7.81 (d, J = 6.2 Hz, 1H), 7.75 (d, J = 8.7 Hz, 1H), 7.69 (m, 1H), 7.43 (s, 1H), 7.23 (s, 2H), 7.15 (d, J = 8.4 Hz, 1H), 6.86 (m, 1H), 5.74 (d, J = 6.1 Hz, 1H), 4.04 (s, 3H), 3.48 (s, 3H), 2.61 (s, 3H), 2.48 (s, 3H). MS (ES+, m/z) 438 (M+H).

### Procedure 3:

To a stirred suspension of the product of Intermediate Example 4 (1.1 g, 3.8 mmol) in 14 mL of MeOH, was added 5-amino-2-methylbenzenesulfonamide (0.78 g, 4.2 mmol, 1.1 equiv) at room temperature. The reaction mixture was heated at reflux for 3 h, then 4 M HCl in 1,4-dioxane (19 µL, 0.076 mmol) was added in one portion. After 4 h, the suspension was cooled to room temperature, and filtered. The resulting solid was washed with 10 mL of MeOH and dried in vacuo to yield 1.3 g (72%) of 5-({4-[(2,3-dimethyl-2H-indazol-6-yl)methylamino]-2-pyrimidinyl}amino)-2-methyl benzenesulfonamide monohydrochloride as a white solid. ¹H NMR (DMSO-d6, 400 MHz) δ 10.95 (s, 1H), 8.36 (s, 1H), 7.86 (d, J = 8.8 Hz, 2H), 7.64-7.59 (m, 2H), 7.40 (m, 3H), 6.93 (dd, J = 8.8, 2.0 Hz, 1H), 5.92 (s, 1H), 4.08 (s, 3H), 3.57 (s, 3H), 2.65 (s, 3H), 2.56 (s, 3H).

### Procedure 4

To a stirred suspension of the product of Intermediate Example 4 (1.1 g, 3.7 mmol) in 10 mL of THF, was added 5-amino-2-methylbenzenesulfonamide (0.70 g, 3.8 mmol, 1.0 equiv) at room temperature. The reaction mixture was heated at reflux for 3 h, then 4 M HCl in 1,4-dioxane (18 µL, 0.072 mmol) was added in one portion. After 5 h, the suspension was cooled to room temperature, and filtered. The resulting solid was washed with 16 mL of THF and dried in the air to yield 1.6 g (92%) of 5-({4-[(2,3-dimethyl-2H-indazol-6-yl)methylamino]-2-pyrimidinyl}amino)-2-methylbenzene sulfonamide monohydrochloride as a light yellow solid.

### Procedure 5

To a stirred suspension of the product of Intermediate Example 4 (1.0 g, 3.6 mmol) in 10 mL of CH₃CN, was added 5-amino-2-methylbenzenesulfonamide (0.70 g, 3.8 mmol, 1.0equiv) at room temperature. The reaction mixture was heated at reflux for 3 h, then 4 M HCl in 1,4-dioxane (18 µL, 0.076 mmol) was added in one portion. After 20 h, the suspension was cooled to room temperature, and filtered. The resulting solid was washed with 10 mL of CH₃CN and dried in the air to yield 1.3 g (73%) of 5-({4-[(2,3-dimethyl-2H-indazol-6-yl)methylamino]-2-pyrimidinyl}amino)-2-methyl benzenesulfonamide monohydrochloride as an off-white solid.

### Procedure 6

To a 2 L jacketed reactor was charged with MeOH (1005 mL), the product of Intermediate Example 4 (84 g, 0.292 mol, 1 equiv) and 5-amino-2-methylbenzenesulfonamide (60 g, 0.320 mol, 1.1 equiv). The solution was stirred and heated to 50 **°**C and 4M HCl in Dioxane (1.46 mL, 2 mol%) was added. The solution was then stirred and heated to reflux with a jacket temperature of 85 **°**C for 10 hours. The resulting slurry was then cooled to 20-25 °C and filtered. The filtered solid was washed with acetonitrile (293 mL X 2) at room temperature. After drying at overnight, under vacuum at 60 **°**C afforded 116 g (81%) of 5-({4-[(2,3-dimethyl-2H-indazol-6-yl)methylamino]-2-pyrimidinyl}amino)-2-methyl benzenesulfonamide monohydrochloride.

### Example 1 b

### Preparation of 5-({4-[(2,3-dimethyl-2H-indazol-6-yl)methylamino]-2-pyrimidinyl}amino)-2-methylbenzenesulfonamide monohydrochloride monohydrate.

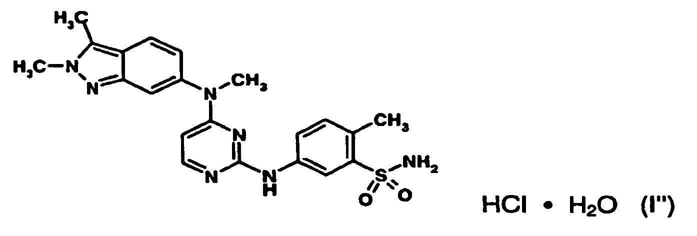

To a round bottom flask, was added 2.6 g of the monohydrochloride salt of Example 1 a, procedure 1, any form. Then added was 39 mL of isopropanol (15 volumes). The mixture was heated to 75 deg C in an oil bath, then 14 mL of 0.05N aqueous HCl (5.4 volumes) was added. The clear solution was cooled to 65 deg C, then seeded with the monohydrate of the monohydrochloride salt of Example 1, procedure 1 (0.05-0.1 wt %). The cloudy solution was stirred at 65 deg C for 60 minutes, then cooled to 0 deg C at ~0.25-0.5 deg C/min. The resulting white solid was filtered and dried to constant weight under vacuum at RT to give 88% yield of 5 -({4-[(2,3-dimethyl-2H-indazol-6-yl)methylamino]-2-pyrimidinyl}amino)-2-methylbenzene sulfonamide monohydrochloride monohydrate.

### Example 1c

### Preparation of 5-({4-[(2,3-dimethyl-2H-indazol-6-y/)methylamino]-2-pyrimidinyl}amino)-2-methylbenzenesulfonamide monohydrochloride anhydrate.

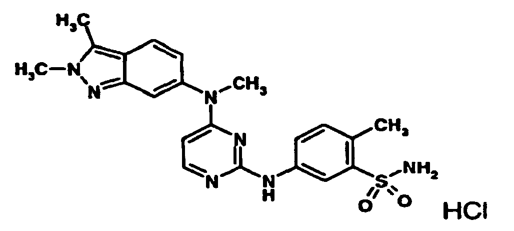

To a 1 L jacketed reactor was charged with acetonitrile (563 mL), water (188 mL) the monohydrochloride salt of Example 1, procedure 6 (50 g, 0.105 mol). The solution was stirred and heated to the jacket temperature at 85**°**C and a clear solution was obtained. The solution was then cooled down to 45°C and held for 90 minutes to cause crystallization of the hydrate After the 90 min hold, the solution was cooled down to 0**°**C, held for an hour and then filtered through a filter-dryer. The filtered solids were then washed with acetonitrile (200 mL X 1) at 0**°**C. The solids were blown in the filter-dryer with nitrogen at 25**°**C until the LOD was less than 25%. Acetonitrile (300 mL) was charged to the solids in filter-dryer, and stirred at 60**°**C for at least 8 hours or until the form conversion was complete (no monohydrate remaining) as observed by DATR to form 5-({4-[(2,3-dimethyl-2H-indazol-6-yl)methylamino]-2-pyrimidinyl}amino)-2-methylbenzenesulfonamide monohydrochloride anhydrate. The contents of the filter-dryer were cooled to ~30**°**C, and the filtrate was pushed off using nitrogen pressure. The filtercake was blown with nitrogen at ~60 **°**C under vacuum until the LOD was less than 0.5%. The contents were cooled to 20 **°**C yielding 37.5 g (75%) of 5-({4-[(2,3-dimethyl-2H-indazol-6-yl)methylamino]-2-pyrimidinyl}amino)-2-methylbenzenesulfonamide monohydrochloride anhydrate.

### Example 2 (REFERENCE EXAMPLE)

### Preparation of N⁴-(2,3-dimethyl-2H-indazol-6-yl)-N⁴-methyl-N²-{4-[(methylsulfonyl)methyl]phenyl}pyrimidine-2,4-diamine.

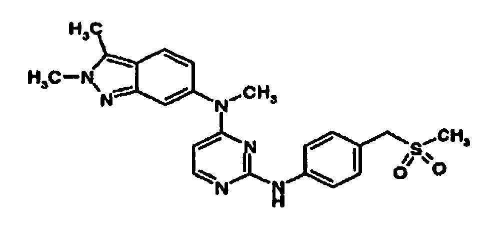

Example 2 was prepared according to the general procedure set forth above in Example 1 using Intermediate Example 4 and the appropriate aniline. The appropriate anilines were prepared using procedures similarly described for Intermediate Examples 5-10. ¹H NMR (300 MHz, d₆DMSO+NaHCO₃) δ 9.37 (bs, 1H), 7.88 (d, *J*= 6.1 Hz, 1H), 7.78 (m, 3H), 7.47 (s, 1H), 7.22 (d, J = 8.5 Hz, 2H), 6.91 (dd, J = 8.8, 1.5 Hz, 1H), 5.84 (d, J = 6.1 Hz, 1H), 4.37 (s, 2H), 4.09 (s, 3H), 3.51 (s, 3H), 2.88 (s, 3H), 2.65 (s, 3H). MS (ES+, m/z) 437 (M+H), 435 (M-H).

### Example 3: (REFERENCE EXAMPLE)

### Preparation of 5-({4-[(2,3-dimethyl-2H-indazol-6-yl)methylamino]-2-pyrimidinyl}amino)-2-methylbenzenesulfonamide monohydrochloride anhydrate.

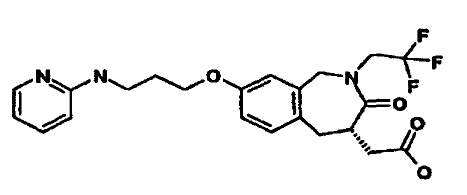

### Preparation 1

### Preparation of methyl (±)-8-hydroxy-2-methyl-3-oxo-2,3,4,5-tetrahydro-1H-2-benzazepine-4-aceta te

### a) 3-[N-(tert-Butoxycarbonyl)-N-methylamino]methyl-4-bromoanisole

40% aqueous methylamine (49 mL, 563 mmole) was added rapidly to a solution of 4-bromo-3-bromomethylanisole (15.76 g, 56.29 mmole) in THF (280 mL) at RT. After 2.5 hr, the reaction was concentrated, and the residue was partitioned between Et₂ O (560 mL) and 1.0 N NaOH (100 mL). The layers were separated, and the organic layer was dried (MgSO₄) and concentrated to a yellow oil: TLC (5% MeOH/CHCl₃) R*_{f}* 0.32. The oil was dissolved in CHCl₃ (280 mL), and di-tert-butyl dicarbonate (1.29 g, 56.29 mmole) was added. The reaction was stirred at RT for 45 min, then was concentrated. Silica gel chromatography (5% EtOAc/toluene) gave the title compound (16.81 g, 90%) as a light yellow oil: TLC (5% EtOAc/toluene) R*_{f}* 0.43; ¹H NMR (400, CDCl₃) mixture of rotamers; 7.42 (d, J=8.7 Hz, 1H, 6.65-6.80 (m, 2 H), 4.40-4.55 (m, 2 H), 3.77 (s, 3 H), 2.81-2.97 (m, 3 H), 1.37-1.60 (m, 9 H); MS (ES) m/e 352/354 (M+Na)⁺

### b) Methyl (±)-3-carbomethoxy-4-[2-[N-(tert-butoxycarbonyl)-N-methylamino]methyl-4 - methoxypheny]butanoate

A solution of 3-[N-(tert-butoxycarbonyl)-N-methylamino]methyl-4-bromoanisole (4.95 g, 15 mmol), dimethyl itaconate (3.08 g, 19.5 mmol), palladium acetate (168 mg, 0.75 mmol), tri-o-tolylphosphine
(457 mg, 1.5 mol), and diisopropylethylamine (5.2 mL, 30 mmol) in propionitrile (75 mL) was heated to reflux for 45 min, then was concentrated on the rotavap. The residue was diluted with Et₂O (150 mL), and the mixture was filtered through celite® to remove insoluble materials. The filtrate was concentrated, and the residue was reconcentrated from xylenes. Chromatography on silica gel (gradient: 20% EtOAc/hexanes, then 1:1 EtOAc/hexanes) removed the phosphine and baseline materials; all other materials with R*_{f}* 0.40-0.70 were collected together and concentrated to leave a cloudy, yellow oil: TLC (30% EtOAc/hexanes) R*_{f}* 0.41 (major product).

The oil was dissolved in MeOH (75 mL), and 10% Pd/C was added carefully. The mixture was shaken under hydrogen (50 psi) for 2.5 hr, then was filtered through celite® to remove the catalyst. The filtrate was concentrated, and the residue was resubmitted to the reaction conditions. After another 2.5 hr, the mixture was filtered through celite® to remove the catalyst, and the filtrate was concentrated to leave a light yellow oil. This was reconcentrated from CHCl₃/hexanes, then was chromatographed on silica gel (gradient: 20% EtOAc/hexanes, then 1:1 EtOAc/hexanes) to afford the title compound (4.53 g, 74%) as a light yellow oil: TLC (30% EtOAc/toluene) R*_{f}* 0.46; ¹H NMR (400, CDCl₃) mixture of rotamers; δ 7.03 (d, J=8.2 Hz, 1 H, 6.65-6.80 (m, 2 H), 4.46 (br s, 2 H), 3.77 (s, 3 H), 3.64 (s, 3 H), 3.63 (s, 3 H), 2.62-3.12 (m, 7 H), 2.35-2.50 (m, 1 H, 1.47 (br s, 9 H); MS (ES) m/e 432 (M+Na) ⁺.

### c) Methyl (±)-3-carbomethoxy-4-[2-(methylamino)methyl-4-methoxyphenyl]butanoate

TFA (55 mL) was added all at once to a solution of methyl (±)-3-carbomethoxy-4-[2-[N-(tert-butoxycarbonyl)-N-(methylamino]methyl- 4-methoxyphenyl]butanoate (4.53 g, 11.06 mmole) in anhydrous CH₂Cl₂ (55 mL) at 0**°**C., and the reaction was warmed to RT. After 1 hr, the reaction was concentrated, and the residue was reconcentrated from toluene (2x100 mL) to leave the title compound (11.06 mmole, quantitative) as a light yellow oil: MS (ES) m/e 310 (M+H) ⁺.

### d) Methyl (±)-8-methoxy-2-methyl-3-oxo-2,3,4,5-tetrahydro-1H-2-benzazepine-4-acet ate

A solution of methyl (±)-3-carbomethoxy-4-[2-(methylamino)methyl-4-methoxyphenyl]butanoate (11.06 mmole) and diisopropylethylamine (5.8 mL, 33.18 mmole) in toluene (110 mL) was heated at reflux for 25 hr, stirred at RT for 4 days, then heated at reflux for another 24 hr. Concentration and silica gel chromatography (5% MeOH in 1:1 EtOAc/CHCl3) gave the title compound (2.88 g, 94%) as a light yellow solid: TLC (5% MeOH in 1:1 EtOAc/CHCl₃) R*_{f}* 0.63; ¹H NMR (250, CDCl₃) δ 7.02 (d, J=8.4 Hz, 1 H, 6.78 (dd, J=8.4, 2.7 Hz, 1 H), 6.63 (d, J=2.7 Hz, 1 H), 5.29 (d, J=16.3 Hz, 1 H), 3.50-3.90 (m, 2 H), 3.79 (s, 3 H), 3.71 (s, 3 H), 2.73-3.16 (m, 3 H), 3.04 (s, 3 H), 2.41 (dd, J=16.7, 5.4 Hz, 1 H; MS (ES) m/e 300 (M+Na) ⁺, 278 (M+H) ⁺.

### e) Methyl (±)-8-hydroxy-2-methyl-3-oxo-2,3,4,5-tetrahydro-1H-2-benzazepine-4-acetate

Anhydrous aluminum chloride (1.35 g, 10.15 mmole) was added all at once to a solution of methyl (±)-8-methoxy-2-methyl-3-oxo-2,3,4,5-tetrahydro-1H-2-benzazepine-4-acet ate (562 mg, 2.03 mmole) and ethanethiol (0.75 mL, 10.15 mmole) in anhydrous CH₂2 Cl₂ (20 mL) at 0**°**C. under argon. The mixture was warmed to RT and stirred for 4.5 hr, then was recooled to 0**°**C. Ice cold H₂O (20 mL) was added, and the mixture was stirred briskly for 5 min, then was extracted with CHCl₃ (3x20 mL). The combined CHCl₃ layers were dried (MgSO₄) and concentrated to leave a residue. The aqueous layer was suction filtered to collect a solid precipitate. This precipitate and the residue from the CHCl₃ layer were combined in 1:1 MeOH/CHCl3, and the solution was concentrated to leave an off-white solid. This was triturated with hot MeOH, and the mixture was allowed to cool to RT. The solid was collected by suction filtration and washed sequentially with cold MeOH and Et₂O. Drying in high vacuum at 40**°** C. gave the title compound (467.9 mg, 88%) as a colorless solid: TLC (5% MeOH/CHCl₃) R*_{f}* 0.17; ¹H NMR (250, DMSO-d₆) δ 9.29(s, 1 H), 6.89 (d, J=8.1 Hz, 1 H), 6.50-6.70 (m, 2 H), 5.16 (d, J=16.4 Hz, 1 H), 3.84 (d, J=16.4 Hz, 1 H), 3.60-3.85 (m, 1 H), 3.56 (s, 3 H), 2.30-3.00 (m, 4 H), 2.86 (s, 3 H); MS (ES) m/e 286 (M+Na) +, 264 (M+H) +.

### Preparation 2

### HPLC Separation of the Enantiomers of methyl (±)-8-hydroxy-3-oxo-2,3,4,5-tetrahydro-1H-2-benzazepine-4-acetate

### a) Methyl (R)-(+)-8-hydroxy-3-oxo-2,3,4,5-tetrahydro-1H-2-benzazepine-4-acetate and methyl (S)-(-)-8-hydroxy-3-oxo-2,3,4,5-tetrahydro-1H-2-benzazepine-4-acetate

Methyl (±)-8-hydroxy-3-oxo-2,3,4,5-tetrahydro-1H-2-benzazepine-4-acetate was resolved into its enantiomers by chiral HPLC using the following conditions: Diacel Chiralpak AS® column (21.2x250 mm), EtOH mobile phase, 7 mL/min flowrate, uv detection at 254 nm, 70 mg injection; t_{R} for methyl (R)-(+)-8-hydroxy-3-oxo-2,3,4,5-tetrahydro-1H-2-benzazepine-4-acetate=21.5 min; t_{R} for methyl (S)-(-)-8-hydroxy-3-oxo-2,3,4,5-tetrahydro-1H-2-benzazepine-4-acetate=39.1 min.

### Preparation 3

### Preparation of (S)-3-oxo-8-[3-(pyridin-2-ylamino)-1-propyloxy]-2-(2,2,2-trifluoroethyl)-2,3,4,5-tetrahydro-1H-2-benzazepine-4-acetic acid

### a) Methyl (S)-3-oxo-8-[3-(1-oxopyridin-2-ylamino)-1-propyloxy]-2-(2,2,2-trifluoroeth yl)-2,3,4,5-tetrahydro-1H-2-benzazepine-4-acetate

To a stirred solution of methyl (S)-8-hydroxy-2-methyl-3-oxo-2,3,4,5-tetrahydro-1H-2-benzazepine-4-acetate (19 g, 57.4 mmol) in dry THF (400 mL) and dry DMF (200 mL) under argon were added 2-(3-hydroxypropylamino)pyridine N-oxide (11.6 g, 69 mmol) and triphenylphosphine (18.0 g, 69 mmol). After all solids had completely dissolved (-30 minutes), the reaction was cooled to 0**°** C. in an ice bath and diisopropyl azodicarboxylate (14.3 mL, 69 mmol) was added via syringe. The reaction was allowed to warm slowly to RT and was stirred for 18 h. Concentration and flash chromatography on silica gel (8:2:1 CHCl₃/EtOAc/EtOH) gave the title compound (20.83 g, 75%) as a solid foam. An additional 5.73 g of product can be obtained by recycling of the recovered starting material from the above reaction to give a total of 26.56 g (96%) of the title compound: MS (ES) m/e 482.2 (M+H)⁺; ¹H NMR (400 MHz, DMSO-d₆) δ 8.09 (dd, J=6.5, 1.3 Hz, 1H), 7.29 (t, 1H), 7.18 (t, 1H), 7.02 (d, J=9.2 Hz, 1H), 6.84-6.79 (m, 3H), 6.59 (t, 1H), 5.32 (d, J=16.5 Hz, 1H), 4.28-4.14 (m, 2H), 4.16 (d, J=16.5 Hz, 1H), 4.02 (t, 2H), 3.84 (m, 1H), 3.58 (s, 3H), 3.40 (dd, 2H), 3.01 (dd, 1H), 2.73 (dd, 1H), 2.70 (dd, 1H), 2.52 (dd, 1H), 2.02 (ddd, 2H).

### b) Methyl (S)-3-oxo-8-[3-(pyridin-2-ylamino)-1-propyloxy]-2-(2,2,2-trifluoroethyl)-2,3,4,5-tetrahydro-1H-2-benzazepine-4-acetate

To a stirred solution of methyl (S)-3-oxo-8-[3-(1-oxopyridin-2-ylamino)-1-propyloxy]-2-(2,2,2-trifluoroethyl)-2,3,4,5-tetrahydro-1H-2-benzazepine-4-acetate (26.56 g, 55 mmol) in isopropanol (500 mL) were added 10% palladium on activated carbon (8 g, 7.5 mmol, carefully pre-wetted in isopropanol under Argon) and cyclohexene (55.7 mL, 550 mmol). The reaction was then heated to reflux under Argon in an oil bath set at 90**°**C. After 6 h an additional amount of 10% palladium on activated carbon (8 g, 7.5 mmol, carefully pre-wetted in isopropanol under Argon) and cyclohexene (55.7 mL, 550 mmol) were added. After an additional 18 h the reaction was hot-filtered through celite®, and the filter pad was washed with 1:1 MeOH/CHCl₃ (400 mL). The filtrate was concentrated under vacuum and the residue was purified by flash chromatography on silica gel (95:5 CHCl₃/MeOH) to give the title compound (19.50 g, 76%) as a white sticky foam: TLC (silica, 5% MeOH in CHCl₃) R*_{f}* 0.52; MS (ES) m/e 466.3 (M+H)⁺ ; ¹H NMR (400 MHz, DMSO-d₆) δ 7.94 (dd, 1H), 7.34 (t, 1H), 7.02 (d, J=9.2 Hz, 1H), 6.81 (m, 2H), 6.54 (t, 1H), 6.46 (m, 2H), 5.31 (d, J=16.5 Hz, 1H), 4.23-4.13 (m, 2H), 4.17 (d, J=16.5 Hz, 1H), 4.02 (t, 2H), 3.82 (m, 1H), 3.58 (s, 3H), 3.36 (m, 2H), 3.01 (dd, 1H), 2.72 (dd, 1H), 2.68 (dd, 1H), 2.50 (dd, 1H), 1.96 (ddd, 2H).

### c) (S)-3-Oxo-8-[3-(pyridin-2-ylamino)-1-propyloxy]-2-(2,2,2-trifluoroethyl)-2,3,4,5-tetrahydro-1H-2-benzazepine-4-acetic acid

To a stirred solution of methyl (S)-3-oxo-8-[3-(pyridin-2-ylamino)-1-propyloxy]-2-(2,2,2-trifluoroethyl)-2,3,4,5-tetrahydro-1H-2-benzazepine-4-acetate (19.50 g, 42 mmol) in dioxane (150 mL) was added aqueous 1 N NaOH (75 mL, 75 mmol). The cloudy reaction was stirred at RT for 2 h, then the resulting homogeneous solution was neutralized with aqueous 1 N HCl (75 mL, 75 mmol). The solution was concentrated to near dryness by rotary evaporation to precipitate out the product. The supernatant was decanted off and the remaining gummy solid was redissolved in methanol. The clear solution was then reconcentrated by rotary evaporation. The remaining solid was triturated with a small volume of water, filtered and dried under vacuum to give the title compound (16.38 g, 86%) as a white powder. HPLC (Hamilton PRP-1®, 25% CH₃CN/H₂O containing 0.1% TFA) k'=3.1; [α]_{D} -112.3**°** (c, 1.0, MeOH); MS (ES) m/e 452.3 (M+H) ⁺; . ¹H NMR (400 MHz, DMSO-d₆) δ 7.95 (dd, 1H), 7.34 (dt, 1H), 7.02 (d, J=9.2 Hz, 1H), 6.81 (m, 2H), 6.58 (t, 1H), 6.47 (m, 2H), 5.30 (d, J=16.5 Hz, 1H), 4.27-4.13 (m, 2H), 4.15 (d, J=16.5 Hz, 1H), 4.02 (t, 1H), 3.78 (m, 1H), 3.37 (m, 2H), 3.00 (dd, 1H), 2.69 (dd, 1H), 2.65 (dd, 1H), 2.41 (dd, 1H), 1.96 (ddd, 2 H). Anal. Calcd for C₂₂H₂₄F₃N₃O₄: C, 58.53; H, 5.36; N, 9.31. Found: C, 58.37; H, 5.42; N, 9.20.

### Biological Data: Effect of the compounds described in Examples 1 and 3 on choroidal neovascularization (CNV) in a mouse model for CNV.

The mice in the following examples were treated in compliance with the ARVO statement for the Use of Animals in Ophthalmic and Vision Research.

### Example 4: Regression Model for CNV

Mice were anesthetized and the pupils were dilated. Bums of krypton laser photocoagulation were delivered to the retina. Administration of the compound described in Example 1 was initiated seven days after the laser-induced injury. Oral doses of either the vehicle alone or vehicle containing the compound of formula (I) (designated as VEGF R in Figure 1) at a dose or 4 mg/kg, 20 mg/kg, or 100 mg/kg were administered twice daily for seven days. After seven day of treatment, the mice were perfused with fluorescein-labeled dextran, and the area of choroidal neovascularization was quantitated. Pazopanib decreased the CNV area in a dose-specific manner. See Figure 1.

### Example 5: Prevention Model for CNV

In this experiment, the compound described in Example 1 (Designated as VEGF R in Figure 2), Example 3 (designated as vitronectin in Figure 2), or a combination of the compounds described in Example 1 and Example 3 (designated as "both" in Figure 2) were administered to each mouse beginning one day before retinal burning was performed according to the methods described in Example 4. The compounds were administered orally twice daily at a dosage of 100 mg/kg for the compound of Example 1 or 45 mg/kg for the compound of Example 3. Fourteen days after the retinal burning, the CNV area was quantitated as described above. The results are shown in Figure 2.

## Claims

1. An oral pharmaceutical formulation comprising a compound of formula (I): 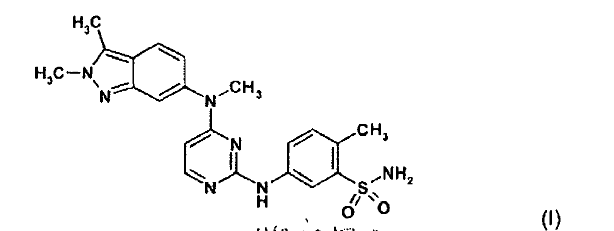 or salt or solvate thereof for use in the treatment of ocular neovascular disorders.

2. An oral formulation for use according to claim 1 wherein said compound is the compound of formula (I'):

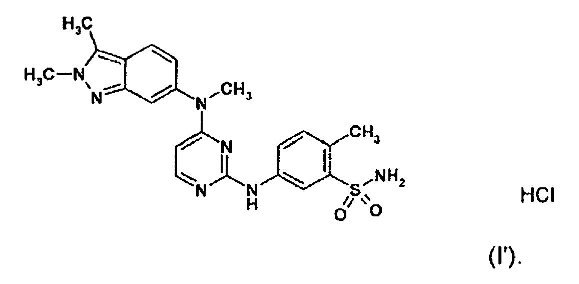

3. An oral formulation for use according to claims 1 and 2 wherein said compound is the compound of formula (I"):

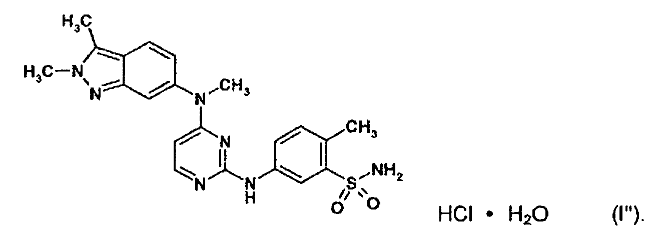

4. An oral formulation for use according to claims 1-3 for administering in conjunction with one or more additional therapeutic agents for the treatment of neovascular disorders.

5. An oral formulation for use according to claim 4 wherein the one or more additional therapeutic agent is pegaptanib and/or ranibizumab.

6. An oral formulation for use according to claims 4-5 wherein the one or more additional therapeutic agents are administered separately.

7. An oral formulation for use according to claim 6 wherein the one or more additional therapeutic agents are administered by different means.

8. An oral formulation for use according to claims 1-7 wherein the ocular neovascular disorder is a choroidal neovascular disorder or a retinal neovascular disorder.

9. An oral formulation for use according to claim 8 wherein the ocular neovascular disorder is exudative age-related macular degeneration (AMD), angiod streaks, pathological myopia, ocular histoplasmosis syndrome, breaks in Bruch's membrane, macular edema (including diabetic macular edema), sarcoidosis, uveitis, atrophic AMD, keratoconus, Sjogren's syndrome, myopia, ocular tumours, corneal graft rejection, corneal injury, neovascular glaucoma, corneal ulceration, corneal scarring, proliferative vitreoretinopathy, retinopathy of prematurity, retinal degeneration, chronic glaucoma, retinal detachment, or sickle cell retinpathy.

10. An oral formulation for use according to claim 9 wherein the ocular neovascular disorder is exudative age-related macular degeneration (AMD).

11. An oral formulation for use according to any of claims 1-10 for prevention of ocular neovascular disorders.

12. An oral formulation for use according to any of claims 1-10 for healing ocular neovascular disorders.

13. An oral formulation for use according to any of claims 1-10 for decreasing the rate of advancement of the disorder.

14. Use of a compound of formula (I):- 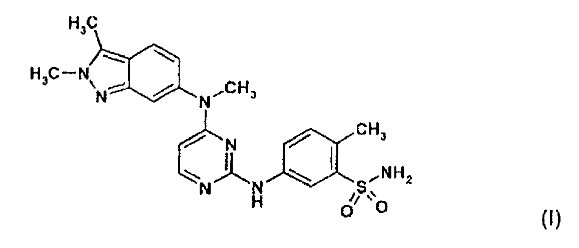 or a salt or solvate thereof in the manufacture of a medicament for oral administration for treatment of ocular neovascular disorders.

15. A compound of formula (I) or a salt or solvate thereof: 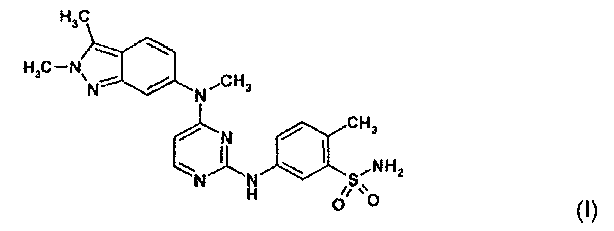 for use in the treatment of ocular neovascular disorders by oral administration.

## Patentansprüche

1. Orale pharmazeutische Formulierung, umfassend eine Verbindung der Formel (I): 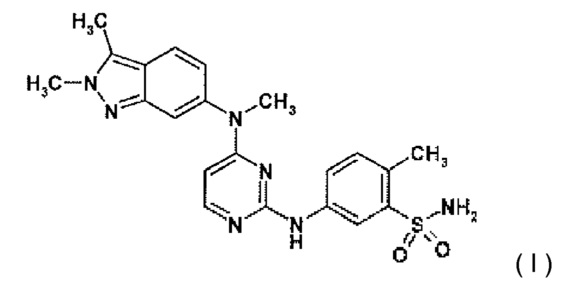 oder ein Salz davon zur Verwendung bei der Behandlung von okularen neovaskulären Störungen.

2. Orale Formulierung zur Verwendung gemäss Anspruch 1, wobei die Verbindung eine Verbindung der Formel (I') ist:

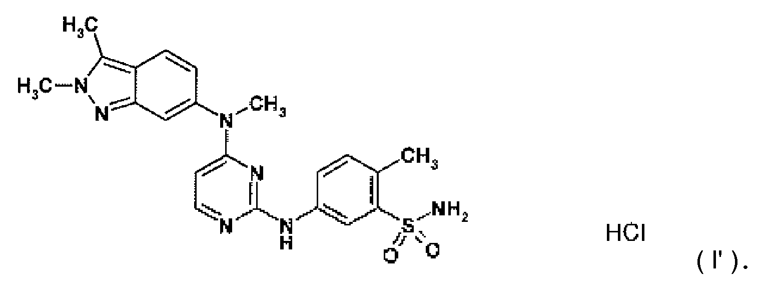

3. Orale Formulierung zur Verwendung gemäss Ansprüchen 1 und 2, wobei die Verbindung eine Verbindung der Formel (I") ist:

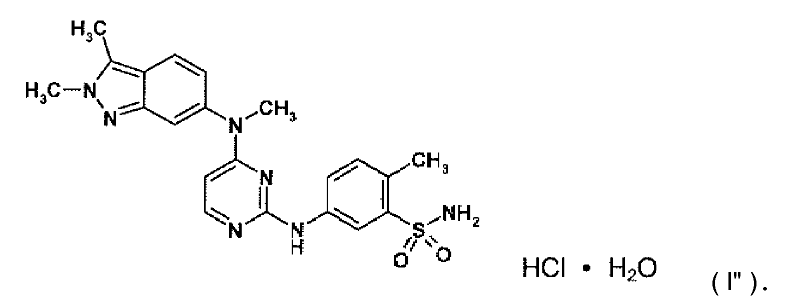

4. Orale Formulierung zur Verwendung gemäss Ansprüchen 1 bis 3 zur Verabreichung in Verbindung mit ein oder mehreren zusätzlichen therapeutischen Mitteln zur Behandlung von neovaskulären Störungen.

5. Orale Formulierung zur Verwendung gemäss Anspruch 4, wobei die ein oder mehreren zusätzlichen therapeutischen Mittel Pegaptanib und/oder Ranibizumab sind.

6. Orale Formulierung zur Verwendung gemäss Ansprüchen 4 bis 5, wobei die ein oder mehreren zusätzlichen therapeutischen Mittel getrennt verabreicht werden.

7. Orale Formulierung zur Verwendung gemäss Anspruch 6, wobei die ein oder mehreren zusätzlichen therapeutischen Mittel auf unterschiedlichen Wegen verabreicht werden.

8. Orale Formulierung zur Verwendung gemäss Ansprüchen 1 bis 7, wobei die okulare neovaskuläre Störung eine choroidale neovaskuläre Störung oder eine retinale neovaskuläre Störung ist.

9. Orale Formulierung zur Verwendung gemäss Anspruch 8, wobei die okulare neovaskuläre Störung eine exudative altersbedingte Makuladegeneration (AMD), gefässähnliche Streifen (angioid streaks), pathologische Myopie, okulares Histoplasmose-Syndrom, Risse in der Bruc-Membran, Makulaödem (einschliesslich diabetisches Makulaödem), Sarkoidose, Uveitis, atrophische AMD, Keratokonus, Sjogren-Syndrom, Myopie, Augentumore, Hornhauttransplantatabstossung, Hornhautverletzung, neovaskuläres Glaukom, Hornhautulzeration, Hornhautnarbenbildung, proliferative Vitreoretinopathie, Frühgeborenen-Retinopathie, Netzhautdegeneration, chronisches Glaukom, Netzhautablösung oder Sichelzellenretinopatie ist.

10. Orale Formulierung zur Verwendung gemäss Anspruch 9, wobei die okulare neovaskuläre Störung exudative altersbedingte Makuladegeneration (AMD)ist.

11. Orale Formulierung zur Verwendung gemäss irgendeinem der Ansprüche 1 bis 10 zur Prävention von okularen neovaskulären Störungen.

12. Orale Formulierung zur Verwendung gemäss irgendeinem der Ansprüche 1 bis 10 zur Heilung von okularen neovaskulären Störungen.

13. Orale Formulierung zur Verwendung gemäss irgendeinem der Ansprüche 1 bis 10 zur Verringerung der Geschwindigkeit des Fortschreitens der Störung.

14. Verwendung einer Verbindung der Formel (I): 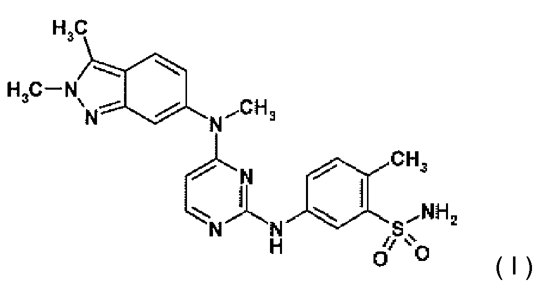 oder eines Salzes davon bei der Herstellung eines Medikaments zur oralen Verabreichung bei der Behandlung von okularen neovaskulären Störungen.

15. Verbindung der Formel (I) oder ein Salz davon: 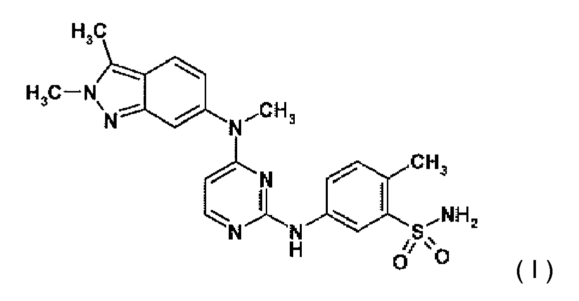 zur Verwendung bei der Behandlung von okularen neovaskulären Störungen durch orale Verabreichung.

## Revendications

1. Formulation pharmaceutique orale comprenant un composé de formule (I) : 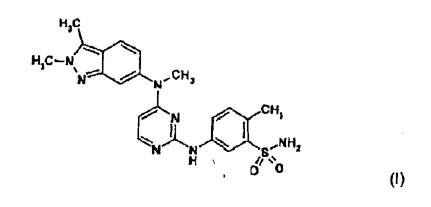 ou un sel de celui-ci pour une utilisation dans le traitement de troubles néovasculaires oculaires.

2. Formulation orale pour une utilisation selon la revendication 1, dans laquelle ledit composé est le composé de formule (I') :

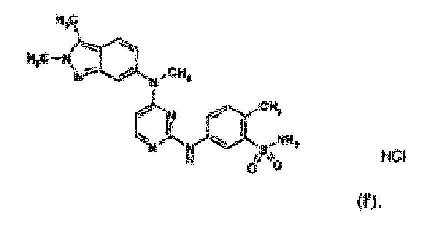

3. Formulation orale pour une utilisation selon les revendications 1 et 2, dans laquelle ledit composé est le composé de formule (I") :

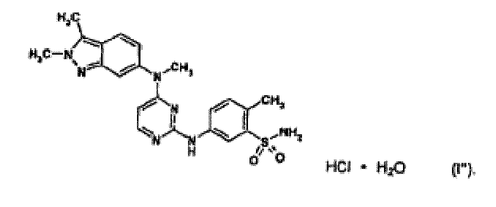

4. Formulation orale pour une utilisation selon les revendications 1 à 3, pour une administration conjointement avec un ou plusieurs agents thérapeutiques supplémentaires pour le traitement de troubles néovasculaires.

5. Formulation orale pour une utilisation selon la revendication 4, dans laquelle le ou les plusieurs agents thérapeutiques supplémentaires sont le pégaptanib et/ou le ranibizumab.

6. Formulation orale pour une utilisation selon les revendications 4 et 5, dans laquelle le ou les plusieurs agents thérapeutiques supplémentaires sont administrés séparément.

7. Formulation orale pour une utilisation selon la revendication 6, dans laquelle le ou les plusieurs agents thérapeutiques supplémentaires sont administrés par des moyens différents.

8. Formulation orale pour une utilisation selon les revendications 1 à 7, dans laquelle le trouble néovasculaire oculaire est un trouble néovasculaire choroïdien ou un trouble néovasculaire rétinien.

9. Formulation orale pour une utilisation selon la revendication 8, dans laquelle le trouble néovasculaire oculaire est la dégénérescence maculaire liée à l'âge (DMLA) exsudative, les stries angioïdes, la myopie pathologique, le syndrome d'histoplasmose oculaire, des ruptures de la membrane de Bruch, l'oedème maculaire (y compris l'oedème maculaire diabétique), la sarcoïdose, l'uvéite, la DMLA atrophique, le kératocône, le syndrome de Sjögren, la myopie, les tumeurs oculaires, le rejet de greffe de la cornée, les lésions cornéennes, le glaucome néovasculaire, l'ulcère de la cornée, la formation de cicatrices cornéennes, la vitréorétinopathie proliférative, la rétinopathie du prématuré, la dégénérescence rétinienne, le glaucome chronique, le décollement de la rétine ou la rétinopathie drépanocytaire.

10. Formulation orale pour une utilisation selon la revendication 9, dans laquelle le trouble néovasculaire oculaire est la dégénérescence maculaire liée à l'âge (DMLA) exsudative.

11. Formulation orale pour une utilisation selon l'une quelconque des revendications 1 à 10, pour la prévention de troubles néovasculaires oculaires.

12. Formulation orale pour une utilisation selon l'une quelconque des revendications 1 à 10, pour la cicatrisation de troubles néovasculaires oculaires.

13. Formulation orale pour une utilisation selon l'une quelconque des revendications 1 à 10, pour la diminution de la vitesse de progression du trouble.

14. Utilisation d'un composé de formule (I) : 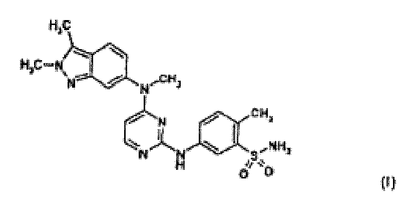 ou d'un sel de celui-ci dans la fabrication d'un médicament destiné à une administration orale pour un traitement de troubles néovasculaires oculaires.

15. Composé de formule (I) ou un sel de celui-ci : 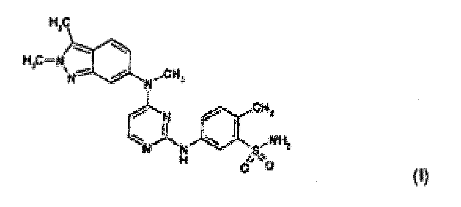 pour une utilisation dans le traitement de troubles néovasculaires oculaires par administration orale.
